# EUROPÄISCHE PATENTSCHRIFT

(11) **EP 2 374 401 B1**
(45) Veröffentlichungstag und Bekanntmachung des Hinweises auf die Patenterteilung: **23.06.2021**
(21) Anmeldenummer: 11159313.3
(22) Anmeldetag: 23.03.2011
(51) Int. Cl.: A61B 1/07, A61B 1/06, A61B 1/04

(54) **Lichtquelleneinrichtung für endoskopische oder exoskopische Anwendungen**
Light source device for endoscopic or exoscopic applications
Dispositif de sources de lumière pour applications endoscopiques et exoscopiques

(30) Priorität: 29.03.2010 DE 102010013307
(43) Veröffentlichungstag der Anmeldung: 12.10.2011
(73) Patentinhaber: Karl Storz SE & Co. KG, 78532 Tuttlingen (DE)
(72) Erfinder: Irion, Klaus M., 78532, Tuttlingen (DE); Hinding, Thomas, 78532, Tuttlingen (DE); Göbel, Werner, 78532, Tuttlingen (DE)
(74) Vertreter: Wimmer, Stephan

(56) Entgegenhaltungen:
- EP-A2- 1 279 364
- EP-A2- 2 020 202
- WO-A1-01/75359
- WO-A1-93/12732
- WO-A1-2004/070844
- DE-A1-102007 015 492
- DE-U1-202005 006 497
- Anonymous: "Lichtquelle - Wikipedia", Wikipedia, 18 March 2010 (2010-03-18), XP055629103, Retrieved from the Internet: URL:https://de.wikipedia.org/w/index.php?t itle=Lichtquelle&oldid=72050653 [retrieved on 2019-10-07]

## Beschreibung

Die vorliegende Erfindung bezieht sich auf eine Lichtquelleneinrichtung mit zumindest zwei Lichtquellen für endoskopische oder exoskopische Anwendungen.

Zur Erzeugung von Licht zur Beleuchtung eines Objekts, das mittels eines Endoskops beobachtet werden soll, werden überwiegend Halogen-Glühlampen und vor allem Gasentladungslampen verwendet, beispielsweise Xenon-Kurzbogenlampen, andere Halid-Kurzbogenlampen und Quecksilberdampf-Hochdrucklampen. Diese Lichtquellen sind herkömmlich mit einer eigenen Leistungsversorgung, einer Steuerung oder Regelung, Filtern und einem Gebläse zur Abfuhr der Abwärme in einer Lichtquelleneinrichtung zusammengefasst. Die Lichtquelleneinrichtung ist als vom eigentlichen Endoskop separate Einheit ausgeführt und mit dem proximalen Ende des Endoskops über ein Lichtleitkabel verbunden.

Nachteile der genannten Lichtquellen betreffen Wirkungsgrad bzw. Leistungsbedarf und Abwärme, Baugröße und Modulierbarkeit. Eine unter diesen Aspekten attraktive Alternative stellen Leuchtdioden dar, insbesondere anorganische Halbleiter-Leuchtdioden, zunehmend auch organische Leuchtdioden. Ein Nachteil einer Leuchtdiode gegenüber einer herkömmlichen Lichtquelle besteht darin, dass nicht nur der von ihr erzeugte Strahlungsfluss Φ, sondern auch die spezifische Ausstrahlung R (in Wm⁻²) und die Strahlungsdichte B = d²Φ/dA dΩcos(θ) (in Wm⁻² sterad⁻¹) kleiner oder deutlich kleiner sind als bei herkömmlichen Lichtquellen. Auch die in das Lichtleitkabel zur Übertragung des Beleuchtungslichts zum Endoskop einkoppelbare Strahlungsleistung ist deshalb bisher geringer.

Das Licht einer herkömmlichen Lichtquelle kann aufgrund der hohen erzielbaren Strahlungsleistung und Intensität gefiltert werden, um beispielsweise Anregungslicht für Autofluoreszenz oder für durch einem Patienten vorher verabreichte 5-Aminolävolinsäure (ALA) induzierte Fluoreszenz von Protoporphyrin IX oder von Fluoreszenz von Indocyaningrün zu erzeugen. Das verwendete Anregungsfilter ist in der Regel nur in einem schmalen Wellenlängenbereich transparent, um ein Überstrahlen des schwachen Fluoreszenzlichts durch remittiertes Anregungslicht zu vermeiden.

Wenn eine herkömmliche Lichtquelle durch eine weiße Leuchtdiode ersetzt wird, verbleibt aufgrund der genannten Nachteile der Leuchtdiode nach einer entsprechenden Filterung nur noch eine geringe Strahlungsleistung im erwünschten schmalen Spektrum des Anregungslichts. Diese geringe Strahlungsleistung reicht typischerweise nicht mehr, um bei den gewünschten Gegenstandsweiten eine ausreichende Beleuchtung zu erzielen bzw. größere Hohlräume ausreichend auszuleuchten. Wünschenswert ist deshalb eine Lichtquelleneinrichtung, bei der ein von der Lichtquelleneinrichtung erzeugtes Spektrum nicht subtraktiv mittels eines Filters, sondern additiv durch Mischung bzw. Kombination des Lichts mehrerer Lichtquellen möglich ist.

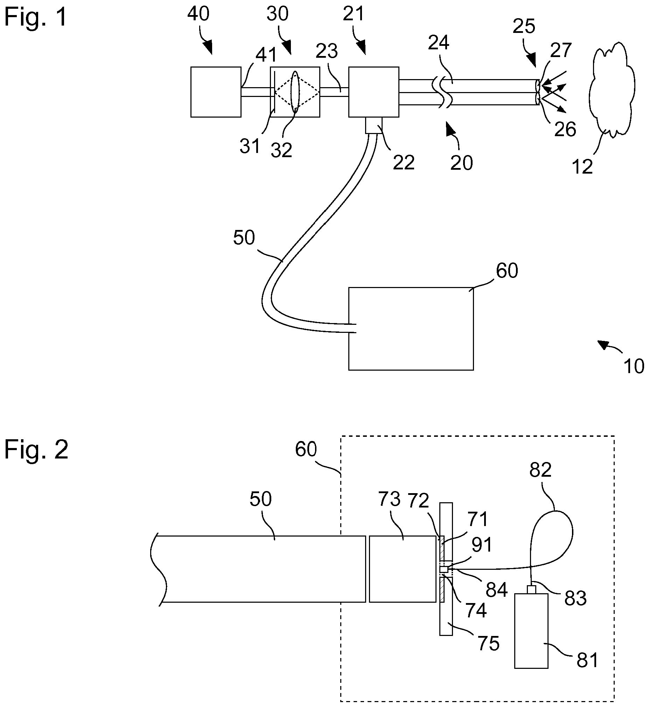

In EP 1 279 364 A3 ist eine Vorrichtung zur bildgebenden Weißlichtdiagnose und zur bildgebenden Fluoreszenzdiagnose von Gewebe beschrieben (Absätze [0001], [0015]). Die Vorrichtung umfasst einen Lichtprojektor 17, dessen Licht über einen Lichtleiter 18 einem Endoskop 5 zugeführt wird (Absatz [0049]). Der Lichtprojektor 17 umfasst ein erstes Leuchtmittel 2 zum Erzeugen von Weißlicht (Absatz [0050]) und einen Diodenlaser (Absatz [0038]) als zweites Leuchtmittel 2a zur Erzeugung von Licht im Fluoreszenzanregungsband des zu untersuchenden Gewebes (Absätze [0038], [0052], Figur 1). Das Licht des Lasers 2a wird mittels eines kleinen Spiegels 10 einem von dem ersten Leuchtmittel ausgehenden Strahlengang 3 überlagert (Absatz [0053]). Das Licht des ersten Leuchtmittels 2 und das Licht des Lasers 2a werden mittels einer Linse 27 in einen Lichtleiter 18 eingekoppelt, der eine Faserbündel umfasst (Absätze [0030], [0037], [0050], [0053], Figur 1).

In DE 10 2007 015 492 A1 ist eine Beleuchtungsvorrichtung 19 für eine Bilderfassungseinrichtung 17 am distalen Ende eines Endoskops 10 beschrieben (Titel, Zusammenfassung, Absätze [0001], [0037], Figuren 1A, 7, 8). Am distalen Ende 20 des Endoskops 10 ist ein Array 63 von Mikro-LEDs 30, 30.x.y angeordnet (Absätze [0037], Figur 1A).

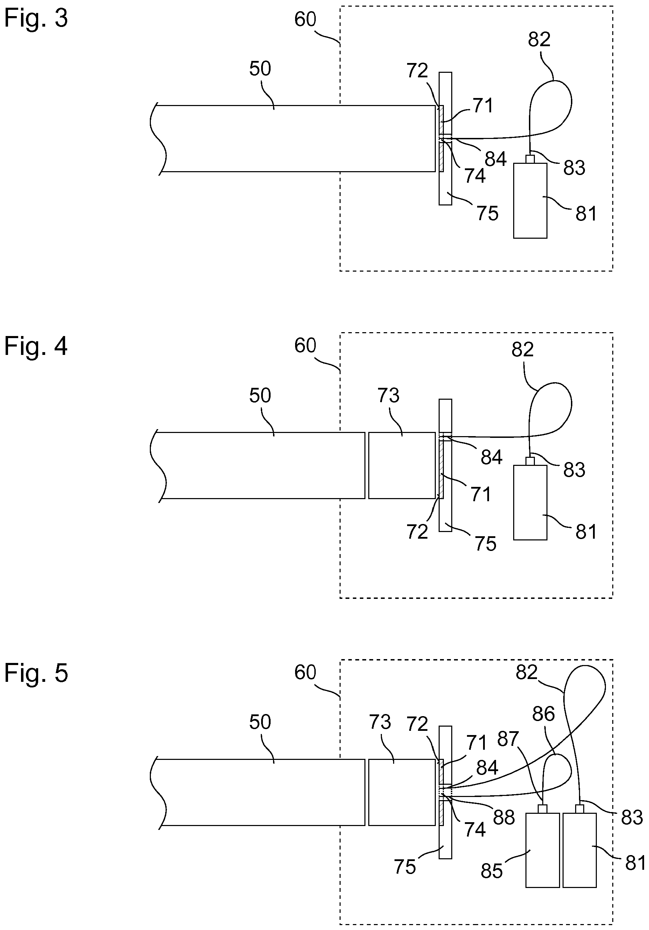

In WO 93/12732 A1 ist eine faseroptische endoskopische Betrachtungsvorrichtung beschrieben, die in ein zahnmedizinisches Handinstrument integriert ist (Seite 10, Zeilen 12 bis 14). Figur 3b zeigt eine mögliche Konfiguration eines optischen Kopfs 24 am distalen Ende des Handinstruments 10 (vgl. Figur 2). Ein erstes, zur Übertragung von Beleuchtungslicht nach distal vorgesehenes optisches Faserbündel 18 umgibt ein zweites, zur Übertragung eines Bilds vorgesehenes optisches Faserbündel (Seite 23, Zeilen 30 bis 32). Das erste, zur Übertragung von Beleuchtungslicht vorgesehene optische Faserbündel 18 endet in einer äußeren kreisförmigen Beleuchtungslinse 53 (Seite 23, Zeile 33, bis Seite 24, Zeile 1). Das zweite, zur Übertragung eines Bilds vorgesehene optische Faserbündel 20 endet in einer inneren ringförmigen Linse 55 (ebd.; die Linie vom Bezugszeichen 55 müsste richtig bis zu dem inneren ringförmigen schraffierten Bereich reichen). Im Zentrum des optischen Kopfs 24 und damit innerhalb der inneren ringförmigen Linse 55 ist ein zentraler Hohlraum 57 vorgesehen, in dem ein "optisches Laserbündel" 59 angeordnet sein kann.

Eine Aufgabe der vorliegenden Erfindung besteht darin, eine Lichtquelleneinrichtung mit einer ersten Lichtquelle und einer zweiten Lichtquelle für endoskopische oder exoskopische Anwendungen zu schaffen.

Diese Aufgabe wird durch den Gegenstand des unabhängigen Anspruchs 1 gelöst.

Weiterbildungen sind in den abhängigen Ansprüchen angegeben.

Eine Lichtquelleneinrichtung für endoskopische oder exoskopische Anwendungen umfasst eine flächige erste Lichtquelle, eine zweite Lichtquelle, einen Beleuchtungsstrahlengang, der dazu ausgebildet ist, ein von der ersten Lichtquelle ausgehendes erstes Lichtbündel für eine endoskopische oder exoskopische Anwendung bereitzustellen, und eine Einkopplungseinrichtung zum Einkoppeln eines zweiten Lichtbündels von der zweiten Lichtquelle in den Beleuchtungsstrahlengang, wobei die Einkopplungseinrichtung so ausgebildet ist, dass am Ort der Einkopplung der Querschnitt des zweiten Lichtbündels kleiner ist als der Querschnitt des ersten Lichtbündels.

Die flächige erste Lichtquelle ist insbesondere insofern flächig, als sie eine ebene oder im wesentlichen ebene lichtemittierende Fläche aufweist. Damit unterscheidet sich die erste Lichtquelle beispielsweise von vielen herkömmlichen Glühlampen und Gasentladungslampen. Deren Glaskolben sind in der Regel zumindest näherungsweise kreiszylindrisch oder rotationselliptisch, insbesondere kugelförmig. Die Glühwendeln selbst sind helikale Strukturen mit stark gekrümmten Oberflächen. Bei einer Gasentladungslampe ist der Raumbereich, in dem die Gasentladung stattfindet, nicht scharf begrenzt, aber jedenfalls in der Regel nicht eben. Ein Beispiel für eine flächige Lichtquelle mit einer insbesondere ebenen lichtemittierenden Fläche ist eine anorganische oder organische Leuchtdiode oder ein Array von Leuchtdioden.

Im Unterschied zu der ersten Lichtquelle ist die zweite Lichtquelle insbesondere punktförmig oder im wesentlichen punktförmig. Dies gilt im unmittelbaren Vergleich mit einer flächigen Lichtquelle wie eine Leuchtdiode beispielsweise für viele Laser, deren Strahlquerschnitt am Auskoppelspiegel sehr klein sein kann.

Die zweite Lichtquelle kann in derselben Einheit oder Baugruppe oder im selben Gerät angeordnet sein wie die erste Lichtquelle. Alternativ ist die zweite Lichtquelle in einer separaten Einheit bzw. einem separaten Gerät angeordnet. Die Lichtquelleneinrichtung kann als von einem Endoskop separate und mit diesem über ein Lichtleitkabel zu verbindende Einheit ausgebildet sein. In diesem Fall umfasst der Beleuchtungsstrahlengang insbesondere das Lichtleitkabel. Der Beleuchtungsstrahlengang kann alternativ oder zusätzlich zu einem Lichtleitkabel ein oder mehrere weitere optische Elemente umfassen, beispielsweise einen Lichtleitkörper, einen Diffusor, eine Kupplung zur lösbaren mechanischen und optischen Kopplung an ein Lichtleitkabel, Linsen, Spiegel, Prismen, Gitter und andere optische Elemente.

Der optionale Lichtleitkörper umfasst insbesondere ein transparentes oder teiltransparentes Material. Mit einem opaken Material kann der Lichtleitkörper gleichzeitig als Diffusor wirken. Der optionale Lichtleitkörper kann als Taper oder in anderer Weise mit einer fokussierenden oder den Querschnitt des Beleuchtungsstrahlengangs reduzierenden Eigenschaft versehen sein.

Alternativ ist die Lichtquelleneinrichtung teilweise oder vollständig in ein Endoskop integriert. Sowohl die erste Lichtquelle als auch die zweite Lichtquelle können jeweils am proximalen Ende oder am distalen Ende des Endoskops angeordnet sein. Wenn die erste Lichtquelle am proximalen Ende eines Endoskops angeordnet ist, umfasst der Beleuchtungsstrahlengang beispielsweise einen Lichtwellenleiter oder ein Bündel von Lichtwellenleitern, welche die erste Lichtquelle mit einem Lichtaustrittsfenster am distalen Ende des Endoskops optisch koppeln.

Wenn die erste Lichtquelle am distalen Ende eines Endoskops angeordnet ist, umfasst der Beleuchtungsstrahlengang beispielsweise lediglich ein Lichtaustrittsfenster aus einem transparenten Material, welches das distale Ende des Endoskops fluiddicht verschließt, und gegebenenfalls einen Raumbereich im Endoskop an dessen distalem Ende zwischen der ersten Lichtquelle und dem Lichtaustrittsfenster. Wenn die erste Lichtquelle am distalen Ende eines Endoskops angeordnet ist und die zweite Lichtquelle am proximalen Ende des Endoskops angeordnet oder mittels eines Lichtleitkabels mit diesem gekoppelt ist, umfasst die Einkopplungseinrichtung beispielsweise einen oder mehrere Lichtwellenleiter, die sich vom proximalen Ende des Endoskops zu dessen distalem Ende erstrecken.

Die Lichtquelleneinrichtung kann zur Bereitstellung von Beleuchtungslicht mit einem beliebigen vorbestimmten Spektrum oder mehreren alternativen vorbestimmten oder diskret oder kontinuierlich veränderbaren Spektren ausgebildet sein. Beispielsweise kann die Lichtquelleneinrichtung zur alternativen Bereitstellung von Beleuchtungslicht mit einem vom menschlichen Auge als weiß wahrgenommenen Spektrum und von Beleuchtungslicht mit einem oder mehreren alternativen Anregungsspektren, die zur Anregung von Fluoreszenz geeignet sind, ausgebildet sein.

Mit Licht wird hier vor allem elektromagnetische Strahlung innerhalb des für das menschliche Auge sichtbaren Spektralbereichs bezeichnet, darüber hinaus aber auch elektromagnetische Strahlung in den angrenzenden Spektralbereichen, insbesondere im Ultravioletten und im Infraroten.

Vom menschlichen Auge als weiß wahrgenommen werden insbesondere Spektren, die keine oder keine größeren Lücken oder andere Ungleichgewichte aufweisen, und denen eine Farbtemperatur zwischen 2500 Kelvin und 6500 Kelvin zugeordnet werden kann. Licht wird als besonders rein weiß wahrgenommen, wenn es eine Farbtemperatur zwischen 3500 Kelvin und 6500 Kelvin aufweist, mehr noch, wenn es eine Farbtemperatur zwischen 4500 Kelvin und 6500 Kelvin aufweist.

Die Lichtquelleneinrichtung kann alternativ oder zusätzlich zur Bereitstellung von Beleuchtungslicht für eine endoskopische Anwendung für die Bereitstellung von Beleuchtungslicht für eine exoskopische Anwendung ausgebildet sein.

Ein Exoskop ist eine zum extrakorporalen Gebrauch vorgesehene und ausgebildete Vorrichtung zur visuellen Inspektion bzw. Betrachtung von Objekten in der Medizin, insbesondere von Objekten an oder nahe äußeren Oberflächen eines menschlichen oder tierischen Körpers. Im Unterschied zu einem Endoskop ist ein Exoskop nicht dazu ausgebildet, um durch eine kleine natürliche oder künstliche Öffnung in einen natürlichen oder künstlichen Hohlraum eingeführt zu werden. Ein Exoskop ist vielmehr für eine Betrachtung eines Objekts ausgebildet, das zumindest während der Betrachtung, insbesondere während einer Operation, von außen sichtbar ist. Entsprechend befindet sich das Exoskop während seines bestimmungsgemäßen Einsatzes teilweise oder vollständig außerhalb des menschlichen oder tierischen Körpers und weist im Unterschied zum Endoskop nicht unbedingt einen langen dünnen Schaft auf.

Ein Exoskop kann eine oder mehrere Kameras oder lichtempfindliche Bildsensoren zur zweidimensionalen oder dreidimensionalen Erfassung und Darstellung, beispielsweise auf einem Bildschirm, ausgebildet sein. Alternativ ist ein Exoskop monokular oder binokular für die unmittelbare Betrachtung mit dem menschlichen Auge ausgebildet. Ein Exoskop ist in der Regel für eine Gegenstandsweite im Bereich von einigen oder wenigen Zentimetern oder wenigen Dezimetern ausgebildet oder optimiert. Ein Exoskop kann eine starke Vergrößerung aufweisen, die eine Auflösung ermöglicht, die mit bloßem Auge nicht erreichbar ist, und damit Eigenschaften einer Lupe bzw. Stereolupe oder eines Mikroskops bzw. Stereomikroskops aufweisen. Vom Mikroskop bzw. Stereomikroskop unterscheidet sich das Exoskop in der Regel durch eine größere Gegenstandsweite.

Der Ort der Einkopplung ist der Ort, ab dem lichtstromabwärts das zweite Lichtbündel im Beleuchtungsstrahlengang und im Wesentlichen in die gleiche Richtung wie das erste Lichtbündel läuft. Wie nachfolgend näher beschrieben wird, wird der Ort der Einkopplung durch ein Ende eines Lichtwellenleiters, aus dem das zweite Lichtbündel austritt, durch eine Linse oder eine Öffnung, durch die das zweite Lichtbündel in den Beleuchtungsstrahlengang eintritt, oder durch einen Spiegel oder ein Gitter gebildet, mittels dessen das zweite Lichtbündel in die Richtung des ersten Lichtbündels umgelenkt wird. Der Ort der Einkopplung ist deshalb in der Regel ein kleiner Raumbereich oder eine ebene oder gekrümmte Fläche, insbesondere eine Grenzfläche oder eine Oberfläche am Ende eines Lichtwellenleiters oder eine Oberfläche einer Linse oder eines Spiegels.

Die Querschnittsflächen des ersten Lichtbündels und des zweiten Lichtbündels sind insbesondere Querschnitte entlang einer Ebene senkrecht zur Hauptausbreitungsrichtung des jeweiligen Lichtbündels, die den Ort der Einkopplung enthält oder schneidet. Sowohl die Querschnittsfläche des ersten Lichtbündels als auch die Querschnittsfläche des zweiten Lichtbündels sind am Ort der Einkopplung insbesondere einfach oder mehrfach zusammenhängend. Eine Querschnittsfläche ist einfach zusammenhängend, wenn sich jede Schleife innerhalb der Querschnittsfläche auf einen Punkt zusammenziehen lässt. Eine Querschnittsfläche ist mehrfach zusammenhängend bzw. n-zusammenhängend, wenn sie - vereinfacht dargestellt - eine oder mehrere Löcher aufweist. Alternativ können jedoch sowohl die Querschnittsfläche des ersten Lichtbündels als auch die Querschnittsfläche des zweiten Lichtbündels jeweils nicht-zusammenhängend sein bzw. aus mehreren voneinander isolierten zusammenhängenden Flächen bestehen.

Ränder von Querschnittsflächen werden beispielsweise von Blenden oder von Rändern von Spiegeln, Linsen, Prismen oder Gittern, von Oberflächen von Lichtwellenleitern oder dem Rand einer Mantelfläche eines Lichtwellenleiters an dessen Ende gebildet. An Orten, an denen der Querschnitt eines Lichtbündels nicht scharf begrenzt ist, wird der Rand des Querschnitts beispielsweise durch die Menge der Punkte definiert, an denen die Intensität 50 % oder einen anderen vorbestimmten Bruchteil der maximalen Intensität beträgt.

Bei einer Lichtquelleneinrichtung, wie sie hier beschrieben ist, ist am Ort der Einkopplung die Querschnittsfläche des zweiten Lichtbündels kleiner, insbesondere wesentlich kleiner, als die Querschnittsfläche des ersten Lichtbündels. Am Ort der Einkopplung beträgt die Querschnittsfläche des zweiten Lichtbündels insbesondere höchstens die Hälfte, ein Fünftel, ein Zehntel, ein Zwanzigstel, ein Fünfzigstel oder ein Hundertstel der Querschnittsfläche des ersten Lichtbündels. Entsprechendes kann für die Inhalte der lichtemittierenden Flächen der Lichtquellen gelten. Der Inhalt der lichtemittierenden Fläche der zweiten Lichtquelle ist insbesondere kleiner oder wesentlich kleiner als der Inhalt der lichtemittierenden Fläche der ersten Lichtquelle, wobei das Verhältnis beispielsweise höchstens 1:2, 1:5, 1:10, 1:20, 1:50 oder 1:100 beträgt.

Die hier beschriebene Lichtquelleneinrichtung ermöglicht beispielsweise eine Kombination zweier Lichtquellen mit unterschiedlicher Abstrahlcharakteristik, insbesondere mit unterschiedlicher Größe der lichtemittierenden Fläche und/oder mit unterschiedlicher Divergenz des emittierten Lichts und eine gleichzeitige oder abwechselnde Einkopplung von deren Licht in den gleichen Beleuchtungsstrahlengang. Beispielsweise kann das Licht einer Leuchtdiode oder eines Arrays von Leuchtdioden mit einer relativ großen lichtemittierenden Fläche mit dem Licht einer Laserdiode oder eines anderen Diodenlasers, das auf eine sehr kleine Fläche gebündelt werden und mit geringen Verlusten in einen Lichtwellenleiter eingekoppelt werden kann, vereinigt bzw. gemischt bzw. kombiniert werden.

Die Lichtquelleneinrichtung ermöglicht ferner eine alternierende Einkopplung von Licht der ersten Lichtquelle und Licht der zweiten Lichtquelle in den Beleuchtungsstrahlengang, ohne den Strahlengang optisch verändern zu müssen. Licht der ersten Lichtquelle und der zweiten Lichtquelle kann deshalb sehr schnell abwechselnd eingekoppelt werden. Dies ermöglicht beispielsweise bei endoskopischen oder exoskopischen Untersuchungen eine von Bild zu Bild wechselnde Beleuchtung, beispielsweise eine alternierende Beleuchtung mit einem Weißlichtspektrum und einem Anregungsspektrum zur Anregung von Fluoreszenz.

Anders als bei einer Vereinigung zweier Lichtstrahlen mittels eines dichroitischen Spiegels oder mittels eines polarisationsabhängig reflektierenden Spiegels ist eine verlustarme Kombination des Lichts zweier Lichtquellen auch dann möglich, wenn diese sich weder in der Wellenlänge noch in der Polarisation unterscheiden. Beispielsweise kann das Licht einer breitbandig emittierenden Leuchtdiode mit dem Licht eines Lasers mit einer Wellenlänge, die innerhalb des Emissionsspektrums der breitbandig emittierenden Leuchtdiode liegt, kombiniert werden. Dies ermöglicht beispielsweise eine Veränderung des von der Leuchtdiode erzeugten Spektrums durch Hinzufügen des Lichts eines Lasers.

Die Einkopplungseinrichtung umfasst in einer Ausführungsform der Erfindung einen Lichtwellenleiter mit einem ersten Ende und einem zweiten Ende, wobei das erste Ende des Lichtwellenleiters mit der zweiten Lichtquelle gekoppelt ist, und wobei das zweite Ende des Lichtwellenleiters eben oder in der lichtemittierenden Fläche der ersten Lichtquelle angeordnet ist. Der Lichtwellenleiter ist beispielsweise ein Single-Mode-Lichtwellenleiter, ein MultiMode-Lichtwellenleiter oder ein Flüssigkeits-Lichtwellenleiter. Die Einkopplungseinrichtung kann einen einzigen derartigen Lichtwellenleiter oder mehrere derartige Lichtwellenleiter in Form eines geordneten oder ungeordneten Bündels umfassen. Das erste Ende des Lichtwellenleiters kann mit der zweiten Lichtquelle lediglich optisch gekoppelt oder auch mechanisch verbunden sein. Wenn das erste Ende des Lichtwellenleiters mit der weiten Lichtquelle mechanisch verbunden ist, kann es zerstörungsfrei lösbar oder nicht zerstörungsfrei lösbar mit der Lichtquelle verbunden sein.

Das zweite Ende des Lichtwellenleiters, genauer die Lichtaustrittsfläche am zweiten Ende des Lichtwellenleiters, ist der bereits erwähnte Ort der Einkopplung. Die lichtemittierende Fläche der ersten Lichtquelle ist eben oder im Wesentlichen eben oder gekrümmt, insbesondere konkav gekrümmt. Die lichtemittierende Fläche der ersten Lichtquelle kann einfach oder mehrfach zusammenhängend oder nicht-zusammenhängend sein. Unten näher beschriebene Ausführungsformen der Erfindung sind eine einzelne Leuchtdiode, eine einzelne Leuchtdiode mit einer Durchgangsbohrung bzw. einem Loch und ein Array von Leuchtdioden. Der Ort der Einkopplung liegt in der lichtemittierenden Fläche oder in einer ebenen oder minimal gekrümmten Fläche, welche die lichtemittierende Fläche der ersten Lichtquelle enthält.

Wenn das zweite Ende des Lichtwellenleiters neben der lichtemittierenden Fläche der ersten Lichtquelle angeordnet ist, ist es unmittelbar neben der lichtemittierenden Fläche oder am Rand der lichtemittierenden Fläche anliegend angeordnet. Dabei ist der Abstand der Mitte des zweiten Endes des Lichtwellenleiters vom Rand der lichtemittierenden Fläche der ersten Lichtquelle insbesondere nicht größer als der Radius oder nicht größer als der Durchmesser oder nicht größer als das Doppelte des Durchmessers des Lichtwellenleiters gegebenenfalls einschließlich seines Mantels (engl.: cladding) und gegebenenfalls einschließlich einer Schutzbeschichtung (engl.: coating oder buffer). Das zweite Ende des Lichtwellenleiters kann an einem geraden oder konkaven Abschnitt des äußeren Rands der Lichtquelle angeordnet sein, insbesondere in einer Einbuchtung bzw. einem Einschnitt des äußeren Rands der ersten Lichtquelle.

Wenn das zweite Ende des Lichtwellenleiters in der lichtemittierenden Fläche der ersten Lichtquelle angeordnet ist, ist es insbesondere in einer Durchgangsbohrung bzw. einem Loch in der mehrfach zusammenhängenden lichtemittierenden Fläche oder an einem konkaven Abschnitt des Rands der lichtemittierenden Fläche angeordnet.

Am Ort der Einkopplung ist typischerweise die lichtemittierende Fläche der ersten Lichtquelle größer oder wesentlich größer als die Querschnittsfläche der Durchgangsbohrung. Das Verhältnis zwischen der lichtemittierenden Fläche der ersten Lichtquelle und der Querschnittsfläche der Durchgangsbohrung beträgt beispielsweise mindestens 2:1, 5:1, 10:1, 20:1, 50:1 oder 100:1. Beispielsweise ist die lichtemittierende Fläche der Leuchtdiode quadratisch oder rechteckig mit einer Seitenlänge zwischen 1 mm und 3 mm, insbesondere mit einer Seitenlänge von ca. 2 mm. Die Durchgangsbohrung kann einen Durchmesser von 100 µm oder weniger, im Einzelfall auch von mehreren hundert µm aufweisen. Die lichtemittierende Fläche ist damit mindestens ca. 100 mal so groß wie die Querschnittsfläche der Durchgangsbohrung.

Ein Vorteil der Verwendung eines Lichtwellenleiters zur Einkopplung des von der zweiten Lichtquelle ausgehenden zweiten Lichtbündels in den Beleuchtungsstrahlengang besteht darin, dass der Lichtwellenleiter mit seinem geringen Querschnitt nur eine kleine Öffnung in der lichtemittierenden Fläche der ersten Lichtquelle benötigt bzw. das Licht der ersten Lichtquelle nur in geringem Maße abschattet bzw. eine Einkopplung des Lichts der zweiten Lichtquelle unmittelbar am Rand der lichtemittierenden Fläche der ersten Lichtquelle ermöglicht. Soweit das Licht der zweiten Lichtquelle verlustarm in die Lichtwellenleiter eingekoppelt werden kann, ermöglicht die Lichtquelleneinrichtung eine effiziente Kombination des Lichts der ersten Lichtquelle und des Lichts der zweiten Lichtquelle.

Bei jeder der genannten Anordnungen des zweiten Endes des Lichtwellenleiters neben oder in der lichtemittierenden Fläche der ersten Lichtquelle ist das zweite Ende des Lichtwellenleiters insbesondere parallel oder im Wesentlichen parallel zur Flächennormale der lichtemittierenden Fläche der ersten Lichtquelle angeordnet.

Bei einer Lichtquelleneinrichtung, wie sie hier beschrieben ist, umfasst die erste Lichtquelle eine Halbleiterlichtquelle mit einem Array von Leuchtdioden oder ein Array von anderen lichtemittierenden Elementen, und der Ort der Einkopplung ist zwischen Leuchtdioden bzw. anderen lichtemittierenden Elementen des Arrays angeordnet. Wenn die Einkopplungseinrichtung wie oben beschrieben einen Lichtwellenleiter umfasst, ist insbesondere das zweite Ende des Lichtwellenleiters zwischen Leuchtdioden oder anderen lichtemittierenden Elementen des Arrays angeordnet. Andernfalls ist beispielsweise eine Linse, ein Gitter oder ein Spiegel vorgesehen, um Licht der zweiten Lichtquelle auf einen Ort zwischen Leuchtdioden bzw. anderen lichtemittierenden Elementen des Arrays, insbesondere auf eine Durchgangsöffnung in einem Substrat des Arrays, zu bündeln. Leuchtdioden des Arrays sind insbesondere weiße Leuchtdioden, die Licht mit einem Spektrum emittieren, das vom menschlichen Auge als weiß wahrgenommen wird. Dazu umfasst das Array von Leuchtdioden beispielsweise eine oder mehrere blaues oder violettes lichtemittierende Halbleiterübergänge und ein phosphoreszierendes oder fluoreszierendes Material, welches das blaue oder violette Licht teilweise absorbiert und grünes, gelbes und/oder rotes Licht emittiert. Alternativ umfasst das Array Leuchtdioden, die Licht mit unterschiedlichen Spektren emittieren, so dass insgesamt ein weißer Farbeindruck entsteht. Beispielsweise umfasst das Array Leuchtdioden, die blaues und gelbes Licht emittieren, oder Leuchtdioden, die blaues, grünes und rotes Licht emittieren.

Leuchtdioden in einem Array weisen in der Regel einen kleinen gegenseitigen Abstand auf, der beispielsweise fertigungstechnisch bedingt und/oder für Leiterbahnen erforderlich ist. Ein solcher Zwischenraum zwischen Leuchtdioden eines Arrays kann ohne Weiteres ausreichen, um dort das Ende eines Lichtwellenleiters anzuordnen. Ein herkömmliches Array von Leuchtdioden muss also unter Umständen nur durch eine Öffnung ergänzt werden.

Bei einer alternativen erfindungsgemäßen Ausführung der Lichtquelleneinrichtung umfasst die erste Lichtquelle eine Leuchtdiode mit einer Öffnung, wobei der Ort der Einkopplung in der Öffnung angeordnet ist.

Die Leuchtdiode kann eine weiße Leuchtdiode sein, wie sie oben beschrieben ist. Die Öffnung reicht insbesondere von einer Rückseite der Leuchtdiode bis zu ihrer lichtemittierenden Fläche, beispielsweise in Form eines geätzten oder gebohrten Durchgangslochs bzw. einer Durchgangsbohrung. Die Öffnung ist insbesondere in der Mitte der lichtemittierenden Fläche der Leuchtdiode angeordnet. Die Leuchtdiode ist so ausgelegt, dass keine elektrische Fehlfunktion, insbesondere kein Kurzschluss, aufgrund der Durchgangsbohrung auftritt. Die Öffnung kann im Verhältnis zur lichtemittierenden Fläche der Leuchtdiode klein sein, insbesondere die lichtemittierende Fläche der Leuchtdiode nur um wenige Prozent, höchstens 10 Prozent oder höchstens 20 Prozent reduzieren. Dies gilt insbesondere wenn das Licht der zweiten Lichtquelle mittels einer Linse, eines Spiegels, eines Gitters oder einer anderen optischen Einrichtung in die Öffnung gebündelt wird. Eine besonders kleine Öffnung ist ausreichend, wenn die Einkopplungseinrichtung einen Lichtwellenleiter umfasst, dessen zweites Ende in der Öffnung angeordnet ist. Die der lichtemittierenden Fläche der Leuchtdiode zugewandte Seite der Öffnung oder das zweite Ende des Lichtwellenleiters bildet den Ort der Einkopplung.

Bei einer Lichtquelleneinrichtung, wie sie hier beschrieben ist, kann die erfindungsgemäße Einkopplungseinrichtung in einer alternativen Ausführungsform zumindest entweder ein Objektiv, einen gekrümmten Spiegel, ein optisches Gitter oder eine andere abbildende Einrichtung umfassen, welche den Querschnitt des zweiten Lichtbündels verringert. Ein Objektiv umfasst eine oder mehrere Linsen und/oder Linsengruppen und/oder einen oder mehrere gekrümmte Spiegel und wirkt insbesondere sammelnd.

Die abbildende Einrichtung ist ausgebildet und angeordnet , um am Rand der lichtemittierenden Fläche der ersten Lichtquelle oder in einer Öffnung in der lichtemittierenden Fläche der ersten Lichtquelle eine Taille bzw. eine Einschnürung bzw. eine Engstelle des zweiten Lichtbündels zu erzeugen. Insbesondere kann die abbildende Einrichtung eine Taille am Ort der Einkopplung erzeugen.

Eine derartige Taillierung bzw. Einschnürung des zweiten Lichtbündels am Ort der Einkopplung ermöglicht eine besonders geringe Störung des Beleuchtungsstrahlengangs, insbesondere eine besonders geringe Minderung der lichtemittierenden Fläche der ersten Lichtquelle oder eine besonders geringe Abschattung des Lichts der ersten Lichtquelle.

Bei einer nicht erfindungsgemäßen Variante der Lichtquelleneinrichtung, wie sie hier beschrieben ist, kann die Einkopplungseinrichtung einen Umlenkspiegel umfassen, der im Beleuchtungsstrahlengang vor der lichtemittierenden Fläche der ersten Lichtquelle angeordnet ist. Der Umlenkspiegel ist insbesondere lichtstromabwärts vor der lichtemittierenden Fläche der ersten Lichtquelle angeordnet. Der Umlenkspiegel bzw. seine reflektierende Fläche bildet den Ort der Einkopplung, ab dem das zweite Lichtbündel im Beleuchtungsstrahlengang und im Wesentlichen in die gleiche Richtung wie das erste Lichtbündel läuft.

Der Umlenkspiegel in dieser nicht erfindungsgemäßen Variante ist kleiner oder wesentlicher kleiner als der Querschnitt des ersten Lichtbündels am Ort des Umlenkspiegels, um einen möglichst geringen Teil des ersten Lichtbündels abzuschatten. Beispielsweise beträgt die auf eine Ebene senkrecht zur Hauptausbreitungsrichtung des ersten Lichtbündels projizierte Fläche des Umlenkspiegels höchstens ein Zehntel, ein Fünftel oder die Hälfte der Querschnittsfläche des ersten Lichtbündels am Ort des Umlenkspiegels. Die Querschnittsfläche des ersten Lichtbündels wird dabei insbesondere in einer Ebene senkrecht zur Hauptausbreitungsrichtung des ersten Lichtbündels gemessen, die den Mittelpunkt, insbesondere den Flächenschwerpunkt, des Umlenkspiegels enthält.

Um die abschattende Wirkung des Umlenkspiegels in dieser nicht erfindungsgemäßen Variante weiter zu mindern, kann der Umlenkspiegel dichroitisch sein. Die Wellenlängenabhängigkeiten des Reflexionsvermögens und des Transmissionsvermögens sind dabei insbesondere so gewählt, dass Licht des zweiten Lichtbündels überwiegend reflektiert und Licht des ersten Lichtbündels überwiegend transmittiert wird.

Die Lichtquelleneinrichtung in dieser nicht erfindungsgemäßen Variante kann insbesondere eine abbildende Einrichtung, die wie oben beschrieben eine Taille des zweiten Lichtbündels erzeugt, und den oben beschriebenen Umlenkspiegel umfassen. In diesem Fall sind die abbildende Einrichtung und der Umlenkspiegel insbesondere so ausgebildet und angeordnet, dass die abbildende Einrichtung die Taille des zweiten Lichtbündels am Ort oder nahe dem Ort des Umlenkspiegels erzeugt. In diesem Fall kann der Umlenkspiegel besonders klein sein.

Der beschriebene Umlenkspiegel in dieser nicht erfindungsgemäßen Variante kann in einen Lichtleitkörper eingebettet sein. Insbesondere kann der Umlenkspiegel in den Lichtleitkörper eingegossen oder im Lichtleitkörper erzeugt sein. Anstelle eines Umlenkspiegels kann beispielsweise ein Gitter vorgesehen sein, das wellenlängenselektiv Licht des zweiten Lichtbündels reflektiert. Ein derartiges Gitter kann beispielsweise auch nach Herstellung eines Lichtleitkörpers in diesem erzeugt werden.

Bei einer weiteren nicht erfindungsgemäßen Variante der Lichtquelleneinrichtung, wie sie hier beschrieben ist, kann der Beleuchtungsstrahlengang mehrere erste Lichtwellenleiter umfassen, deren erste Enden mit der ersten Lichtquelle gekoppelt sind, und die Einkopplungseinrichtung zumindest einen zweiten Lichtwellenleiter umfassen, dessen erstes Ende mit der zweiten Lichtquelle gekoppelt ist, wobei zweite Enden der ersten Lichtwellenleiter und zweite Enden des zumindest einen zweiten Lichtwellenleiters parallel und nebeneinander an einer Fläche angeordnet sind. Die Fläche, an der die zweiten Enden der ersten Lichtwellenleiter und des zumindest einen zweiten Lichtwellenleiters angeordnet sind, kann in dieser nicht erfindungsgemäßen Variante eben oder gekrümmt sein und bildet insbesondere den Ort der Einkopplung des zweiten Lichtbündels in den Beleuchtungsstrahlengang. Wenn die ersten Lichtwellenleiter und der zumindest eine zweite Lichtwellenleiter bereits eine Strecke lichtstromaufwärts der Fläche parallel zueinander und nebeneinander angeordnet sind, ist der Ort der Einkopplung der Ort, ab dem die ersten Lichtwellenleiter und der zumindest eine zweite Lichtwellenleiter nebeneinander und parallel zueinander angeordnet sind.

Eine Lichtquelleneinrichtung mit dem Beleuchtungsstrahlengang, der mehrere erste Lichtwellenleiter und zumindest einen zweiten Lichtwellenleiter umfasst, ist ein nicht erfindungsgemäßes Beispiel, bei dem die Querschnittsfläche des ersten Lichtbündels mehrere nicht-zusammenhängende Einzelflächen umfasst, nämlich die Querschnittsflächen der einzelnen lichtführenden Kerne der Lichtwellenleiter. Im Fall mehrerer zweiter Lichtwellenleiter ist auch die Querschnittsfläche des zweiten Lichtbündels nicht-zusammenhängend und umfasst die Querschnittsflächen der lichtführenden Kerne der zweiten Lichtwellenleiter.

Ein Vorteil eines Beleuchtungsstrahlengangs in dieser nicht erfindungsgemäßen Variante mit mehreren ersten Lichtwellenleitern und zumindest einem zweiten Lichtwellenleiter besteht darin, dass diese Lichtwellenleiter gleichzeitig Bestandteil eines Lichtleitkabels von der separaten Lichtquelleneinrichtung zum proximalen Ende des Endoskops und/oder vom proximalen Ende zum distalen Ende des Endoskops sein können.

Im Falle mehrerer zweiter Lichtwellenleiter in dieser nicht erfindungsgemäßen Variante können diese zufällig oder quasizufällig oder regelmäßig angeordnet sein. Die zweiten Lichtwellenleiter sind insbesondere entsprechend einem ein- oder zweidimensionalen Punktgitter angeordnet. Dadurch kann das Licht des zweiten Lichtbündels in nahezu jeder beliebigen erwünschten Weise im Querschnitt des Beleuchtungsstrahlengangs verteilt werden.

Bei einer Lichtquelleneinrichtung, wie sie hier beschrieben ist, kann die erste Lichtquelle zur Erzeugung eines ersten Spektrums mit einer ersten Halbwertsbreite ausgebildet, und die zweite Lichtquelle zur Erzeugung eines zweiten Spektrums mit einer zweiten Halbwertsbreite ausgebildet sein, wobei die zweite Halbwertsbreite nicht mehr als die Hälfte der ersten Halbwertsbreite beträgt.

Die erste Lichtquelle ist insbesondere zur Erzeugung eines breiten Spektrums im für das menschliche Auge sichtbaren Spektralbereich und/oder in angrenzenden Spektralbereichen (Infrarot, Ultraviolett) ausgebildet. Insbesondere kann die erste Lichtquelle ausgebildet sein, um ein vom menschlichen Auge als weiß wahrgenommenes Spektrum zu erzeugen. Wenn die erste Lichtquelle eine Halbleiterlichtquelle mit einer oder mehreren Leuchtdioden umfasst, können jede einzelne Leuchtdiode oder gegebenenfalls alle Leuchtdioden zusammen zur Emission eines breiten Spektrums, insbesondere eines weißen Spektrums ausgebildet sein.

Die zweite Lichtquelle kann demgegenüber zur Erzeugung eines schmalen Spektrums ausgebildet sein. Dies gilt beispielsweise, wenn die zweite Lichtquelle eine Laserdiode, einen Diodenlaser oder einen anderer Laser umfasst. Das schmale Spektrum der zweiten Lichtquelle kann ebenfalls ganz oder teilweise innerhalb oder außerhalb des für das menschliche Auge sichtbaren Spektralbereichs liegen. Das schmale Spektrum der zweiten Lichtquelle kann ganz oder teilweise mit dem breiten Spektrum der ersten Lichtquelle überlappen.

Die Addition bzw. Mischung des breiten Spektrums und des schmalen Spektrums ermöglicht beispielsweise eine abwechselnde oder gleichzeitige Beobachtung eines Objekts in remittiertem Weißlicht und in Fluoreszenzlicht. Alternativ kann durch Mischung des ersten Spektrums und des zweiten Spektrums eine Verbesserung der Spektralcharakteristik erzielt werden, beispielsweise eine Verbesserung der Farbwiedergabe.

Bei einer Lichtquelleneinrichtung, wie sie hier beschrieben ist, kann die zweite Lichtquelle eine Laserdiode oder einen anderen Diodenlaser oder einen anderen Laser umfassen.

Die Laserdiode kann insbesondere als Pigtail ausgeführt sein, wobei das Licht der Laserdiode unmittelbar oder mittels einer Linse oder eines Gitters oder einer anderen abbildenden Einrichtung in ein erstes Ende eines Lichtwellenleiters eingekoppelt wird. Das erste Ende des Lichtwellenleiters kann mit der Laserdiode zerstörungsfrei trennbar oder nicht zerstörungsfrei trennbar verbunden, insbesondere mit ihr verkittet sein.

Ein Vorteil der Verwendung eines Lasers besteht darin, dass das von ihm emittierte Licht eine besonders hohe Intensität, insbesondere eine hohe Strahlungsleistung bei einem geringen Querschnitt aufweist. Überdies weist das von einem Laser emittierte Licht eine besonders geringe Divergenz auf. Das Licht eines Lasers kann deshalb besonders gut gebündelt werden, beispielsweise in eine kleine Öffnung in einer Leuchtdiode oder einem Array von Leuchtdioden oder auf einen kleinen Umlenkspiegel. Aus dem gleichen Grund kann das Licht eines Lasers besonders effizient in einen Lichtwellenleiter eingekoppelt werden. Ein besonderer Vorteil einer Pigtail-Laserdiode besteht in der effizienten und robusten Ankopplung des Lichtwellenleiters an die Laserdiode.

Laserdioden, Diodenlaser und andere Laser emittieren ferner in der Regel nur eine oder wenige Wellenlängen. Aufgrund dieses schmalbandigen Spektrums kann ihr Licht beispielsweise mittels eines wellenlängenselektiv reflektierenden Spiegels oder mittels eines Gitters eingekoppelt werden, der bzw. das außerhalb eines schmalen Wellenlängenbereichs transparent ist und Licht der ersten Lichtquelle nicht abschattet. Ferner können Laserdioden, Diodenlaser und andere Laser in der Regel schnell ein- und ausgeschaltet und beispielsweise gepulst betrieben werden.

Bei einer Lichtquelleneinrichtung, wie sie hier beschrieben ist, kann die erste Lichtquelle in einem Endoskop oder in einem Exoskop und die zweite Lichtquelle separat von dem Endoskop bzw. Exoskop angeordnet sein.

Die zweite Lichtquelle kann in diesem Fall über einen dünnen und hoch flexiblen Lichtwellenleiter mit dem Endoskop bzw. mit dem Exoskop gekoppelt werden. Dieser Lichtwellenleiter kann mit einer Leistungsversorgungsleitung zur Versorgung der ersten Lichtquelle im Endoskop mit elektrischer Leistung in einem Kabel integriert werden.

### Kurzbeschreibung der Figuren

Nachfolgend werden Ausführungsformen anhand der beigefügten Figuren näher erläutert. Es zeigen:
- Figur 1: eine schematische Darstellung eines Endoskopiesystems;
- Figur 2: eine schematische Darstellung einer Lichtquelleneinrichtung;
- Figur 3: eine schematische Darstellung einer weiteren Lichtquelleneinrichtung;
- Figur 4: eine schematische Darstellung einer weiteren Lichtquelleneinrichtung;
- Figur 5: eine schematische Darstellung einer weiteren Lichtquelleneinrichtung;
- Figur 6: eine schematische Darstellung einer weiteren Lichtquelleneinrichtung;
- Figur 7: eine schematische Darstellung einer nicht erfindungsgemäßen Lichtquelleneinrichtung;
- Figur 8: eine schematische Darstellung einer weiteren Lichtquelleneinrichtung;
- Figur 9: eine schematische Darstellung einer weiteren Lichtquelleneinrichtung;
- Figur 10: eine schematische Darstellung einer weiteren nicht erfindungsgemäßen Lichtquelleneinrichtung;
- Figur 11: eine schematische Darstellung einer weiteren nicht erfindungsgemäßen Lichtquelleneinrichtung;
- Figur 12: eine schematische Darstellung einer weiteren Lichtquelleneinrichtung;
- Figur 13: eine schematische Darstellung einer weiteren nicht erfindungsgemäßen Lichtquelleneinrichtung;
- Figur 14: eine schematische Darstellung einer weiteren Lichtquelleneinrichtung;
- Figur 15: eine schematische Darstellung einer weiteren Lichtquelleneinrichtung;
- Figur 16: eine schematische Darstellung eines Endoskops;
- Figur 17: eine schematische Darstellung eines weiteren Endoskops.

### Beschreibung der Ausführungsformen

Figur 1 zeigt eine schematische Darstellung eines Endoskopiesystems 10 zur Betrachtung bzw. optischen Erfassung eines Objekts 12. Das Endoskopiesystem 10 umfasst ein Endoskop 20. Am proximalen Ende 21 des Endoskops 20 sind eine Kupplung 22 für ein Lichtleitkabel und eine Kupplung 23 für eine Kamera angeordnet. Vom proximalen Ende 21 erstreckt sich ein starrer oder flexibler Schaft 24 zu einem distalen Ende 25 des Endoskops 20. Am distalen Ende 25 weist das Endoskop 20 ein Lichtaustrittsfenster 26 und ein Lichteintrittsfenster 27 auf.

Über die Kupplung 23 ist das Endoskop 20 mit einer Kamera 30 gekoppelt. Die Kameras 30 umfasst einen lichtempfindlichen Bildsensor 31 und ein Objektiv 32. Abweichend von der Darstellung in Figur 1 und alternativ dazu kann anstelle der Kamera 30 ein Okular vorgesehen sein, durch das ein vom Endoskop 20 erfasstes Bild des Objekts 12 unmittelbar mit dem menschlichen Auge beobachtet werden kann.

Über die Kupplung 22 ist das proximale Ende 21 des Endoskops 20 mittels eines Lichtleitkabels 50 mit einer Lichtquelleneinrichtung 60 gekoppelt. Die Lichtquelleneinrichtung 60 ist zur Erzeugung von Beleuchtungslicht mit einem oder mehreren vorbestimmten oder veränderbaren Beleuchtungsspektren ausgebildet. Verschiedene Varianten der Lichtquelleneinrichtung 60 werden unten mit Bezug auf die Figuren 2 bis 13 beschrieben.

Abweichend von der Darstellung in Figur 1 und alternativ zu dieser können die Komponenten des Endoskopiesystems 10 teilweise oder vollständig integriert sein. Beispielsweise können die Kamerasteuerung 40 oder Teile derselben in die Kamera 30 integriert sein. Die Kamera 30 oder die Kamera 30 und die Kamerasteuerung 40 können in das Endoskop 20 integriert sein, beispielsweise an dessen proximalem Ende 21. Die Lichtquelleneinrichtung 60 kann teilweise oder vollständig in das Endoskop 20 integriert sein, insbesondere an dessen proximalem Ende und/oder an dessen distalem Ende.

Zur endoskopischen Erfassung des Objekts 12 mittels des Endoskopiesystems 10 erzeugt die Lichtquelleneinrichtung 60 Licht, das in das Lichtleitkabel 50 eingekoppelt und von diesem zum proximalen Ende 21 des Endoskops 20 übertragen wird. Mittels eines in Figur 1 nicht dargestellten Strahlengangs, insbesondere mittels eines oder mehrerer Lichtwellenleiter, wird das von der Lichtquelleneinrichtung 60 erzeugte Beleuchtungslicht vom proximalen Ende 21 des Endoskops 20 zu dessen distalem Ende 25 übertragen. Das Beleuchtungslicht tritt am Lichtaustrittsfenster 26 aus dem Endoskop 20 aus und fällt auf das Objekt 12. Das Beleuchtungslicht kann vom Objekt 12 absorbiert, reflektiert oder gestreut werden. Ferner kann das Beleuchtungslicht abhängig von seinen spektralen Eigenschaften und den Eigenschaften der Oberfläche des Objekts 12 Fluoreszenz hervorrufen.

Vom Objekt 12 ausgehendes reflektiertes oder gestreutes Beleuchtungslicht oder Fluoreszenzlicht fällt auf das Lichteintrittsfenster 27 am distalen Ende 25 des Endoskops 20. Dieses Licht wird über einen in Figur 1 nicht dargestellten Beobachtungsstrahlengang vom distalen 25 des Endoskops 20 zu dessen proximalem Ende 21 und weiter zur Kamera 30 oder zu einem in Figur 1 nicht dargestellten Okular übertragen. Der Beobachtungsstrahlengang umfasst beispielsweise eine Stablinsenoptik oder ein geordnetes Bündel von Lichtwellenleitern. Im Fall der in Figur 1 dargestellten Verwendung einer Kamera erzeugt ein Objektiv 32, das zumindest eine Linse, einen gekrümmten Spiegel oder eine andere abbildende Einrichtung umfasst, ein reelles Bild am lichtempfindlichen Bildsensor 31 der Kamera 30.

Nachfolgend werden anhand der Figuren 2 bis 13 erfindungsgemäße Ausführungsformen und weitere, nicht erfindungsgemäße Beispiele der Lichtquelleneinrichtung 60 beschrieben. Ein Gehäuse bzw. eine Begrenzung der Lichtquelleneinrichtung 60 ist dabei jeweils nur in einer unterbrochenen Linie dargestellt. Insbesondere wenn die Lichtquelleneinrichtung 60 so, wie durch die unterbrochene Linie angedeutet, eine Einheit bildet, kann sie weitere Komponenten umfassen. Zu den weiteren Komponenten zählen beispielsweise eine Leistungsversorgung zur Versorgung von Lichtquellen und anderen Komponenten der Lichtquelleneinrichtung 60 mit Leistung, insbesondere mit elektrischer Leistung, und eine Steuerung oder Regelung zur Steuerung von Lichtquellen oder anderen Komponenten der Lichtquelleneinrichtung 60.

Lichtemittierende Flächen, Lichtleitkabel, Lichtleitkörper und andere optische Elemente sind teilweise beabstandet voneinander dargestellt, um zu verdeutlichen, dass sie zumindest ursprüngliche getrennt hergestellte und/oder funktional getrennte Komponenten darstellen. Tatsächlich können diese optischen Komponenten abweichend von den Darstellungen in den Figuren 2 bis 13 aneinander angrenzen, insbesondere flächig aneinander anliegen, oder beispielsweise miteinander verkittet sein. Dadurch können beispielsweise Verluste durch Reflexionen an Grenzflächen vermieden oder zumindest verringert werden.

Figur 2 zeigt eine Lichtquelleneinrichtung 60 mit einer Leuchtdiode oder einem Array von Leuchtdioden 71. Nachfolgend wird zunächst davon ausgegangen, dass die Lichtquelleneinrichtung 60 eine einzelne Leuchtdiode 71 aufweist.

Die Leuchtdiode 71 weist eine lichtemittierende Fläche 72 auf, die eben oder im Wesentlichen eben ist. Die Leuchtdiode 71 kann eine anorganische oder organische Leuchtdiode sein. Die Leuchtdiode 71 kann einen lichtemittierenden Halbleiterübergang, der zur Emission von blauem oder violettem Licht ausgebildet ist, und eine phosphoreszierende oder fluoreszierende Schicht an der lichtemittierenden Oberfläche 72 aufweisen. Die phosphoreszierende oder fluoreszierende Schicht an der lichtemittierenden Fläche 72 kann ausgebildet sein, um einen Teil des blauen oder violetten Lichts zu absorbieren und durch Fluoreszenz oder Phosphoreszenz Licht im grünen, gelben und/oder roten Spektralbereich zu emittieren. Mit diesem Aufbau kann die Leuchtdiode 71 ausgebildet sein, um Licht mit einem breiten Spektrum zu emittieren, insbesondere vom menschlichen Auge als weiß empfundenes Licht. Vom menschlichen Auge als weiß empfunden wird Licht insbesondere, wenn ihm eine Farbtemperatur zwischen 3500 Kelvin und 6500 Kelvin, manchmal auch schon ab 2500 Kelvin zugeordnet werden kann, und wenn der Farbwiedergabeindex nicht zu niedrig ist, insbesondere mindestens 50, 70 oder 80 beträgt.

Der lichtemittierenden Fläche 72 der Leuchtdiode 71 gegenüber ist ein Lichtleitkörper 73 angeordnet. Der Lichtleitkörper kann, wie in Figur 2 angedeutet, von der lichtemittierenden Oberfläche 72 der Leuchtdiode 71 beabstandet sein. Alternativ kann der Lichtleitkörper 73 flächig an der lichtemittierenden Fläche 72 der Leuchtdiode 71 anliegen oder mit dieser verkittet sein. Die der lichtemittierenden Fläche 72 der Leuchtdiode 71 gegenüberliegende Fläche des Lichtleitkörpers 73 ist Lichteintrittsfläche, die von der lichtemittierenden Fläche 72 der Leuchtdiode 71 abgewandte Fläche des Lichtleitkörpers 73 ist Lichtaustrittsfläche. Die Lichteintrittsfläche und die Lichtaustrittsfläche des Lichtleitkörpers 73 können durch eine Beschichtung entspiegelt sein, insbesondere wenn sie von der lichtemittierenden Oberfläche 72 der Leuchtdiode bzw. von anderen optischen Elementen beabstandet sind. Weitere Oberflächen des Lichtleitkörpers 73 können verspiegelt sein.

Die Leuchtdiode 71 weist eine Öffnung 74 in Form eines Durchgangslochs, das sich von der lichtemittierenden Fläche 72 bis zu einer gegenüberliegenden Rückseite der Leuchtdiode 71 erstreckt, auf. Die Leuchtdiode 71 ist an einem Kühlkörper 75 angeordnet, der eine mit der Öffnung 74 der Leuchtdiode 71 korrespondierende Öffnung aufweist. Aufgrund der Öffnung 74 ist die lichtemittierende Fläche 72 der Leuchtdiode 71 nicht einfach, sondern mehrfach zusammenhängend bzw. weist ein entsprechendes Loch auf.

Die Lichtquelleneinrichtung 60 umfasst ferner eine Laserdiode 81 und einen Lichtwellenleiter 82. Ein erstes Ende 83 des Lichtwellenleiters 82 ist mit der Laserdiode 81 optisch gekoppelt, insbesondere mit der lichtemittierenden Fläche der Laserdiode 81 verkittet. Ein zweites Ende 84 des Lichtwellenleiters 82 ist gegenüber einer Linse 91 angeordnet oder, wie in Figur 2 dargestellt, berührt die Linse 91. Das zweite Ende 84 des Lichtwellenleiters 82 kann mit der Linse 91 verkittet sein. Die Linse 91 ist in oder an der Öffnung 74 in der Leuchtdiode 71 angeordnet. Die Linse 91 ist beispielsweise eine Gradientenindexlinse. Die von dem Lichtwellenleiter 82 abgewandte Oberfläche der Linse 91 ist parallel zur lichtemittierenden Oberfläche 72 der Leuchtdiode 71 angeordnet und liegt insbesondere in einer Ebene oder im Wesentlichen in einer Ebene mit der lichtemittierenden Fläche 72 der Leuchtdiode 71. Die Linse 91 ermöglicht eine Formung des vom zweiten Ende 84 des Lichtwellenleiters 82 ausgehenden zweiten Lichtbündels, insbesondere eine Beeinflussung von dessen Divergenz.

Gegenüber der Lichtaustrittsfläche des Lichtleitkörpers 73 kann ein Ende des bereits oben anhand der Figur 1 beschriebenen Lichtleitkabels 50 angeordnet werden. Der Lichtleitkörper 73 oder der Lichtleitkörper 73 und das Lichtleitkabel 50 gemeinsam bilden einen Beleuchtungsstrahlengang, der dazu ausgebildet ist, von der Leuchtdiode 71 ausgehendes Licht, insbesondere ein von der Leuchtdiode 71 ausgehendes erstes Lichtbündel für eine endoskopische oder exoskopische Anwendung bereitzustellen, insbesondere zur Beleuchtung eines Objekts 12 und/oder zur Anregung von Fluoreszenz am Objekt 12.

Die Ausdehnung des von der Leuchtdiode 71 erzeugten ersten Lichtbündels und seine Querschnittsfläche sind im Wesentlichen durch die lichtemittierende Fläche 72 der Leuchtdiode 71 und die Querschnitte des Lichtleitkörpers 73 und ggf. des Lichtleitkabels 50 bestimmt. Die Hauptausbreitungsrichtung des von der Leuchtdiode 71 erzeugten ersten Lichtbündels im Beleuchtungsstrahlengang entspricht im Wesentlichen den Flächennormalen der lichtemittierenden Fläche 72 der Leuchtdiode 71, der Lichteintrittsfläche und der Lichtaustrittsfläche des Lichtleitkörpers und der Längsachse bzw. Längsrichtung des Lichtleitkabels 50.

Von der Laserdiode 81 erzeugtes Licht wird am ersten Ende 83 in den Lichtwellenleiter 82 eingekoppelt, von diesem zu dessen zweitem Ende 84 übertragen und mittels der Linse 91 in den Lichtleitkörper 73 und damit in den Beleuchtungsstrahlengang eingekoppelt. Der Ort der Einkopplung des von der Laserdiode 81 erzeugten zweiten Lichtbündels in den Beleuchtungsstrahlengang ist die Öffnung 74 in der Leuchtdiode 71, insbesondere derjenige Abschnitt der durch die lichtemittierende Fläche 72 der Leuchtdiode 71 definierten Ebene, der innerhalb der Öffnung 74 liegt. Im weiteren Sinne können auch die Lichtaustrittsfläche der Linse 91 oder der der Öffnung 74 in der Leuchtdiode 71 gegenüberliegende Abschnitt der Lichteintrittsfläche des Lichtleitkörpers 73 als Ort der Einkopplung angesehen werden.

Am Ort der Einkopplung weist das von der Laserdiode 81 erzeugte und vom Lichtwellenleiter 82 übertragene zweite Lichtbündel eine Querschnittsfläche auf, die höchstens der Querschnittsfläche der Öffnung 74 in der Leuchtdiode 71 entspricht. Am gleichen Ort bzw. in der gleichen Ebene entspricht die Querschnittsfläche des von der Leuchtdiode 71 ausgehenden ersten Lichtbündels im Wesentlichen der lichtemittierenden Fläche 72 der Leuchtdiode 71. Damit ist die Querschnittsfläche des von der Laserdiode 81 erzeugten zweiten Lichtbündels wesentlich kleiner als die Querschnittsfläche des von der Leuchtdiode 71 erzeugten ersten Lichtbündels. Ab dem Ort der Einkopplung lichtstromabwärts sind die Hauptrichtungen des von der Leuchtdiode 71 ausgehenden ersten Lichtbündels und des von der Laserdiode erzeugten zweiten Lichtbündels gleich oder im Wesentlichen gleich. Die Lichtbündel können sich jedoch in ihrer Divergenz deutlich unterscheiden.

Wenn die Leuchtdiode 71, wie oben beschrieben, eine weiße Leuchtdiode ist, ist die Laserdiode 81 beispielsweise zur Emission von blauem, violettem, rotem oder anderem Licht zur Anregung von Fluoreszenz am Objekt 12 ausgebildet. Die Leuchtdiode 71 und die Laserdiode 81 werden beispielsweise alternierend betrieben, um das Objekt 12 abwechselnd mit Weißlicht und mit Fluoreszenz-Anregungslicht zu bestrahlen. Auch eine gleichzeitige Bestrahlung bzw. Beleuchtung des Objekts 12 mit Weißlicht und mit Fluoreszenz-Anregungslicht ist möglich.

Alternativ ist die Laserdiode 81 beispielsweise ausgebildet, um grünes, gelbes oder rotes Licht zu emittieren, welches das von der Leuchtdiode 71 emittierte Spektrum ergänzt. Dazu werden die Leuchtdiode 71 und die Laserdiode 81 insbesondere gleichzeitig betrieben. Mit einem gleichzeitigen Betrieb der Leuchtdiode 71 und der Laserdiode 81 ist auch ein Betrieb gemeint, bei dem die Leuchtdiode 71 und die Laserdiode 81 nur teilweise gleichzeitig oder so schnell alternierend Licht emittieren, dass die unterschiedlichen Beleuchtungszustände vom menschlichen Auge bzw. von der Kamera 30 nicht mehr zeitlich aufgelöst werden können. Beispielsweise werden die Leuchtdiode 71 und die Laserdiode 81 so schnell alternierend betrieben, dass innerhalb des Belichtungsintervalls eines einzigen von der Kamera 30 erfassten Bilds oder Halbbilds das Objekt 12 einmal oder mehrmals mit Licht der Leuchtdiode 71 und einmal oder mehrmals mit Licht der Laserdiode 81 beleuchtet wird.

Durch die Ergänzung des von der Leuchtdiode 71 emittierten Lichts durch das von der Laserdiode 81 emittierte Licht kann das Spektrum des das Objekt 12 beleuchtenden Beleuchtungslichts so verbessert werden, dass ein besserer Farbeindruck entsteht, insbesondere ein natürlicher Farbeindruck. Dies geht insbesondere einher mit einer Erhöhung des Farbwiedergabeindex des Spektrums des Beleuchtungslichts.

Wie bereits erwähnt, kann die Lichtquelleneinrichtung 60 anstelle einer einzelnen Leuchtdiode 71 auch ein Array von Leuchtdioden mit einer ein- oder zweidimensionalen regelmäßigen oder unregelmäßigen Anordnung von Leuchtdioden umfassen. In diesem Fall kann die Linse 91 in einem Zwischenraum zwischen zwei Leuchtdioden angeordnet sein, wobei das Bezugszeichen 74 anstelle einer Öffnung in einer Leuchtdiode einen Zwischenraum zwischen zwei Leuchtdioden bezeichnet. Auch bei den nachfolgend anhand der Figuren 3 bis 14 dargestellten Lichtquelleneinrichtungen kann jeweils anstelle einer Leuchtdiode ein Array von Leuchtdioden vorgesehen sein.

Figur 3 zeigt eine Lichtquelleneinrichtung 60 ähnlich der oben anhand der Figur 2 dargestellten Lichtquelleneinrichtung. Die Lichtquelleneinrichtung 60 unterscheidet sich von der oben anhand der Figur 2 dargestellten Lichtquelleneinrichtung insbesondere dadurch, dass kein Lichtleitkörper 73 vorgesehen ist. Ferner weist die Lichtquelleneinrichtung 60 keine Linse in der Öffnung 74 in der Leuchtdiode 71 auf. Beide Unterschiede sind unabhängig voneinander. Dies gilt weitgehend auch für die nachfolgend anhand der weiteren Figuren beschriebenen Ausführungsformen.

Bei dem in Figur 3 gezeigten Verzicht auf den Lichtleitkörper 73 wird der Beleuchtungsstrahlengang zunächst im Wesentlichen durch den Raum vor der lichtemittierenden Fläche 72 der Leuchtdiode 71 oder, genauer, durch den Raum, in den die Leuchtdiode 71 Licht einer Mindest-Intensität emittiert, gebildet. Wenn das Lichtleitkabel 50 in die Lichtquelleneinrichtung 60 eingesetzt ist oder ständig mit ihr verbunden ist, wird der Beleuchtungsstrahlengang, ähnlich wie oben anhand der Figur 2 erläutert, beispielsweise durch die Mantelfläche des lichtleitenden Kerns des Lichtwellenleiters 50 begrenzt.

Der Verzicht auf einen Lichtleitkörper reduziert die Anzahl der Grenzflächen, an denen störende Reflexionen stattfinden können, und reduziert die Herstellungskosten. Die gleichen Vorteile gelten unabhängig auch für den Verzicht auf die in Figur 2 gezeigte Linse 91 am zweiten Ende 84 des Lichtwellenleiters 82. Durch einen Verzicht auf eine Linse in der Öffnung 74 der Leuchtdiode 71 wird der Raumbedarf verringert, und die Öffnung 74 in der Leuchtdiode 71 muss - anders als in den Figuren dargestellt - nur einen unwesentlich größeren Querschnitt aufweisen als der Lichtwellenleiter 82.

Figur 4 zeigt eine schematische Darstellung einer weiteren Lichtquelleneinrichtung 60, die in einigen Merkmalen den oben anhand der Figuren 2 und 3 dargestellten Lichtquelleneinrichtungen ähnlich ist. Die Lichtquelleneinrichtung 60 unterscheidet sich von der oben anhand der Figur 2 dargestellten dadurch, dass das zweite Ende 84 des Lichtwellenleiters 82 nicht in einer Öffnung 74, sondern am Rand der Leuchtdiode 71 angeordnet ist. Das zweite Ende 84 des Lichtwellenleiters 82 kann an einem geraden oder konkaven Abschnitt des äußeren Rands der Leuchtdiode 71 angeordnet sein, insbesondere in einer Einbuchtung bzw. einem Einschnitt des äußeren Rands der ersten Lichtquelle.

Die in Figur 4 dargestellte Anordnung bietet sich beispielsweise an, wenn die Leuchtdiode 71 bzw. deren lichtemittierende Fläche 72 rechteckig und der Querschnitt des Lichtleitkörpers 73 kreisförmig ist. Allgemein bietet sich die in Figur 4 dargestellte Anordnung an, wenn der Querschnitt des Lichtleitkörpers 73 an mindestens einer Stelle über die lichtemittierende Fläche 72 der Leuchtdiode 71 hinaus ragt.

Ein weiterer Unterschied der Lichtquelleneinrichtung 60 von der oben anhand der Figur 2 dargestellten besteht darin, dass, ähnlich wie bei der oben anhand der Figur 3 dargestellten Lichtquelleneinrichtung, am zweiten Ende 84 des Lichtwellenleiters 82 keine Linse vorgesehen ist. Damit können die bereits oben anhand der Figur 3 dargestellten Vorteile erzielt werden.

Die beiden genannten Unterschiede der Lichtquelleneinrichtung 60 von der oben anhand der Figur 2 dargestellten Lichtquelleneinrichtung sind wiederum weitgehend unabhängig voneinander. Insbesondere kann auch bei einer Anordnung des zweiten Endes 84 des Lichtwellenleiters 82 an einem äußeren Rand der Leuchtdiode 71 eine Linse vorgesehen sein. Auch bei der oben anhand der Figur 3 dargestellten Lichtquelleneinrichtung ohne Lichtleitkörper kann das zweite Ende 84 des Lichtwellenleiters 82 am Rand der Leuchtdiode 71 angeordnet sein.

Ein Vorteil der Anordnung des zweiten Endes 84 des Lichtwellenleiters 82 am Rand der Leuchtdiode 71 besteht darin, dass diese keine Öffnung aufweisen muss. Die Herstellungskosten der Leuchtdiode 71 können deshalb geringer sein.

Figur 5 zeigt eine schematische Darstellung einer Lichtquelleneinrichtung 60, die in einigen Merkmalen der oben anhand der Figur 2 dargestellten Lichtquelleneinrichtung ähnlich ist. Die Lichtquelleneinrichtung 60 unterscheidet sich von der oben anhand der Figur 2 dargestellten Lichtquelleneinrichtung insbesondere darin, dass zwei Laserdioden 81, 85 mit zwei Lichtwellenleitern 82, 86 vorgesehen sind. Ein erstes Ende 83 des ersten Lichtwellenleiters 82 ist mit der ersten Laserdiode 81 gekoppelt. Ein erstes Ende 87 des zweiten Lichtwellenleiters 86 ist mit der zweiten Laserdiode 85 gekoppelt. Zweite Ende 84, 88 der beiden Lichtwellenleiter 82, 86 sind in einer Öffnung 74 in der Leuchtdiode 71 angeordnet. Alternativ können die zweiten Ende 84, 88 der Lichtwellenleiter 82, 86 in zwei von einander beabstandeten Öffnungen in der Leuchtdiode 71 angeordnet sein.

Die beiden Laserdioden 81, 85 können zur Emission von Licht mit dem gleichen oder zwei verschiedenen Spektren ausgebildet sein. Wenn beide Laserdioden 81, 85 das gleiche Spektrum emittieren, kann durch die Einkopplung des Lichts zweier Laserdioden deren Strahlungsleistung addiert werden. Wenn die Laserdioden 81, 85 Licht mit unterschiedlichen Wellenlängen emittieren, kann deren Licht zur alternativen oder gleichzeitigen Anregung verschiedener Fluoreszenzen oder zur Verbesserung bzw. Korrektur des von der Leuchtdiode 71 erzeugten Spektrums eingesetzt werden.

Bei der Lichtquelleneinrichtung 60 sind anders als bei der oben anhand der Figur 2 dargestellten Lichtquelleneinrichtung und ähnlich den oben anhand der Figuren 3 und 4 dargestellten Lichtquelleneinrichtungen keine Linsen an den zweiten Enden 84, 88 der Lichtquellenleiter 82, 86 vorgesehen. Dies kann die bereits oben anhand der Figuren 3 und 4 dargestellten Vorteile geringerer Herstellungskosten und eines geringeren Raumbedarfs in der Öffnung 74 haben. Alternativ können bei der Lichtquelleneinrichtung 60 ähnlich wie bei der oben anhand der Figur 2 dargestellten Lichtquelleneinrichtung eine oder zwei Linsen an den zweiten Enden 84, 88 der Lichtwellenleiter 82, 86 vorgesehen sein.

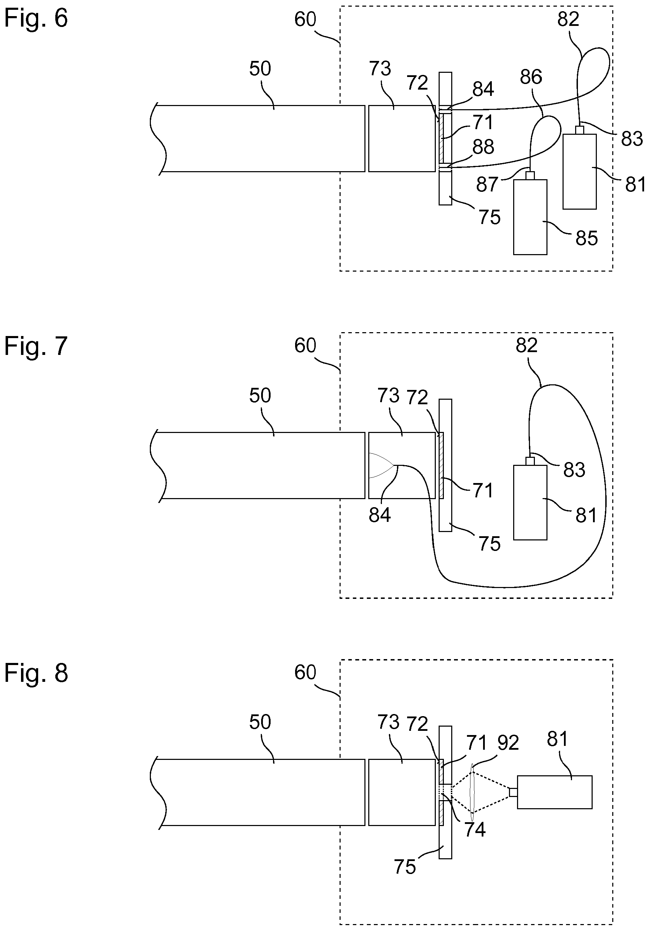

Figur 6 zeigt eine schematische Darstellung einer Lichtquelleneinrichtung 60, die in mehreren Merkmalen den oben anhand der Figuren 2 und 5 dargestellten Lichtquelleneinrichtungen ähnlich ist. Ähnlich wie die oben anhand der Figur 5 dargestellten Lichtquelleneinrichtung sind zwei Laserdioden 81, 85 mit zwei Lichtwellenleitern 82, 86 vorgesehen. Anders als bei der oben anhand der Figur 5 dargestellten Lichtquelleneinrichtung sind die zweiten Enden 84, 88 der Lichtwellenleiter 82, 86 jedoch nicht in einer Öffnung, sondern am Rand der Leuchtdiode 71 angeordnet. Insbesondere sind die zweiten Enden 84, 88 der Lichtwellenleiter 82, 86 an gegenüberliegenden Abschnitten des Rands der Leuchtdiode 71 angeordnet. Diese gegenüberliegenden Abschnitte können gerade oder gekrümmt sein, insbesondere konkav.

Vorteile dieser Anordnung und weitere Merkmale können denen der oben anhand der Figuren 4 bzw. 5 beschriebenen Lichtquelleneinrichtungen entsprechen. Ähnlich wie bei den oben anhand der Figuren 3 bis 5 dargestellten Lichtquelleneinrichtungen sind keine Linsen an den zweiten Enden 84, 88 der Lichtwellenleiter 82, 86 vorgesehen. Abweichend von der Darstellung in Figur 6 können jedoch ähnlich wie bei den anderen oben beschriebenen Lichtquelleneinrichtungen und deren Varianten Linsen an den zweiten Enden 84, 88 der Lichtwellenleiter 82, 86 vorgesehen sein.

Bei den oben anhand der Figuren 5 und 6 dargestellten Lichtquelleneinrichtungen kann das Licht einer Laserdiode mittels mehrerer Lichtwellenleiter, deren zweite Enden in mehreren verschiedenen Öffnungen 74 oder an verschiedenen Stellen am Rand der Leuchtdiode 71 angeordnet sind, in den Beleuchtungsstrahlengang eingekoppelt werden. Anstelle von zwei Laserdioden 81, 85, deren Licht mittels zweier Lichtwellenleiter 82, 86 in den Beleuchtungsstrahlengang eingekoppelt wird, kann das Licht von drei oder mehr Laserdioden über eine entsprechende Anzahl von Lichtwellenleitern in den Beleuchtungsstrahlengang eingekoppelt werden.

Die oben anhand der Figuren 4 bis 6 dargestellten Lichtquelleneinrichtungen weisen jeweils einen Lichtleitkörper auf, wie er bereits oben anhand der Figur 2 dargestellt wurde. Abweichend davon und alternativ dazu können die oben anhand der Figuren 4 bis 6 dargestellten Lichtquelleneinrichtungen jeweils ohne einen Lichtleitkörper ausgebildet sein. In diesem Fall kann das Lichtleitkabel 50 lösbar oder permanent (beispielsweise mittels eines optisch transparenten Kitts) mit der lichtemittierenden Fläche 72 der Leuchtdiode 71 und den zweiten Enden 84, 88 der Lichtwellenleiter 82, 86 bzw. daran angeordneten Linsen optisch gekoppelt oder optisch gekoppelt und mechanisch verbunden sein.

Ein Lichtleitkörper 73 kann zur Homogenisierung bzw. Mischung der von der Leuchtdiode 71 und der oder den Laserdioden 81, 85 erzeugten Lichtbündel beitragen. Dies gilt bei einer Anordnung des oder der zweiten Enden 84, 88 von Lichtwellenleitern 82, 86 in Öffnungen 74 und insbesondere bei einer Anordnung des oder der zweiten Enden 84, 88 am Rand der Leuchtdiode 71. Dazu weist der Lichtleitkörper 73 beispielsweise leicht opake Eigenschaften auf.

Figur 7 zeigt eine schematische Darstellung einer nicht erfindungsgemäßen Variante der Lichtquelleneinrichtung 60, die in einigen Merkmalen den oben anhand der Figuren 2 bis 6 dargestellten Lichtquelleneinrichtung ähnlich ist. Die Lichtquelleneinrichtung 60 unterscheidet sich von der oben anhand der Figur 2 dargestellten Lichtquelleneinrichtung insbesondere darin, dass das zweite Ende 84 des Lichtwellenleiters 82 nicht in einer Öffnung in der Leuchtdiode 71, sondern lichtstromabwärts vor der lichtemittierenden Fläche 72 der Leuchtdiode 71 angeordnet ist. Das zweite Ende 84 des Lichtwellenleiters 82 ist dazu insbesondere in dem Lichtleitkörper 73 angeordnet, beispielsweise in diesen eingegossen. Das zweite Ende 84 des Lichtwellenleiters 82 ist parallel zur Flächennormale der lichtemittierenden Fläche 72 der Leuchtdiode 71 und damit parallel zur an diesem Ort vorliegenden Hauptstrahlungsrichtung der Leuchtdiode 71 angeordnet.

Bei der Lichtquelleneinrichtung 60 stellt die Stirnfläche am zweiten Ende 84 des Lichtwellenleiters 82 den Ort der Einkopplung des von der Laserdiode 81 erzeugten zweiten Lichtbündels in den an dieser Stelle im Wesentlichen durch den Lichtleitkörper 73 definierten Beleuchtungsstrahlengang dar. Aufgrund der Ausrichtung des zweiten Endes 84 des Lichtwellenleiters 82 sind am Ort der Einkopplung die Hauptstrahlungsrichtungen des von der Leuchtdiode 71 ausgehenden ersten Lichtbündels und des von der Laserdiode 81 erzeugten zweiten Lichtbündels gleich. Abweichend von der Darstellung in Figur 7 kann am zweiten Ende 84 des Lichtwellenleiters 82 eine kleine Linse zur Formung des am zweiten Ende 84 aus dem Lichtwellenleiter 82 austretenden zweiten Lichtbündels vorgesehen sein.

Abweichend von der Darstellung in Figur 7 kann der Lichtwellenleiter 82 nicht seitlich sondern durch eine Öffnung in der Leuchtdiode 72 in den Lichtleitkörper 73 führen. In diesem Fall ist die Anordnung beispielsweise ähnlich wie oben anhand der Figuren 2 und 5 dargestellt, wobei aber das zweite Ende 84 des Lichtwellenleiters 82 über die lichtemittierende Fläche 72 lichtstromabwärts übersteht und in den Lichtleitkörper 73 hinein ragt. Der Ort der Einkopplung ist in diesem Fall die Stirnfläche am zweiten Ende 84 des Lichtwellenleiters 82.

Figur 8 zeigt eine schematische Darstellung einer Lichtquelleneinrichtung 60, die in einigen Merkmalen den oben anhand der Figuren 2 bis 6 dargestellten Lichtquelleneinrichtung ähnlich ist. Die Lichtquelleneinrichtung 60 unterscheidet sich von der oben anhand der Figur 2 dargestellten Lichtquelleneinrichtung insbesondere dadurch, dass anstelle eines Lichtwellenleiters ein Objektiv 92, das insbesondere eine oder mehrere Linsen umfasst, vorgesehen ist. Die Laserdiode 81 und das Objektiv 92 sind relativ zu der Leuchtdiode 71 und der Öffnung 74 in der Leuchtdiode 71 so angeordnet, dass das von der Laserdiode 81 erzeugte zweiten Lichtbündel in der Öffnung 74 eine Taille bzw. eine Einschnürung aufweist.

Der Ort der Einkopplung des von der Laserdiode 81 erzeugten zweiten Lichtbündels in den im Wesentlichen durch die lichtemittierende Fläche 72 und den Querschnitt des Lichtleitkörpers 73 begrenzten Beleuchtungsstrahlengang ist die der lichtemittierenden Fläche 72 der Leuchtdiode 71 zugewandte Seite der Öffnung 74. Wenn der Lichtleitkörper 73 unmittelbar an der lichtemittierenden Fläche 72 der Leuchtdiode 71 anliegt, ist auch der an die Öffnung 74 angrenzende Abschnitt der Lichteintrittsfläche des Lichtleitkörpers 73 Ort der Einkopplung.

Zur Formung des von der Laserdiode 81 erzeugten zweiten Lichtbündels am Ort des Eintritts in den Beleuchtungsstrahlengang kann in der Öffnung 74 abweichend von Figur 8 eine Linse oder ein Objektiv vorgesehen sein. Auch bei der Lichtquelleneinrichtung 60 kann - ähnlich wie bei den oben anhand der Figuren 2 bis 6 dargestellten Lichtquelleneinrichtungen bzw. deren Varianten - der Lichtleitkörper 73 entfallen. In diesem Fall liegt abweichend von Figur 8 die Lichteintrittsfläche des Lichtleitkabels 50 der lichtemittierenden Oberfläche 72 der Leuchtdiode 71 und der Öffnung 74 in der Leuchtdiode 71 mit einem (insbesondere kleinen) Abstand gegenüber. Alternativ liegt die Lichteintrittsfläche des Lichtleitkabels 50 an der lichtemittierenden Fläche 72 der Leuchtdiode 71 unmittelbar an oder ist mit ihr verkittet.

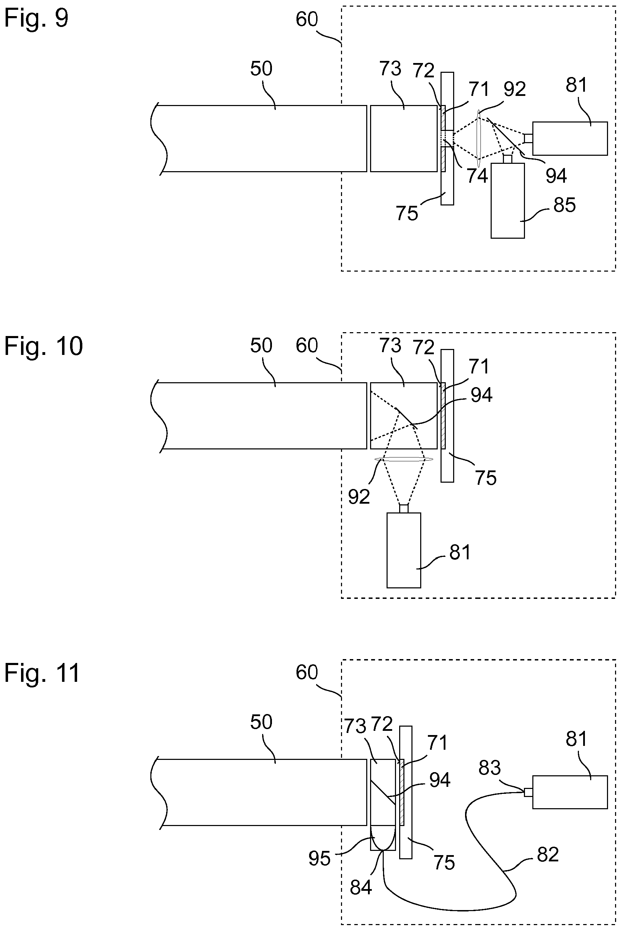

Figur 9 zeigt eine schematische Darstellung einer Lichtquelleneinrichtung 60, die in mehreren Merkmalen der oben anhand der Figur 8 dargestellten Lichtquelleneinrichtung ähnlich ist. Die Lichtquelleneinrichtung 60 unterscheidet sich von der oben anhand der Figur 8 dargestellten Lichtquelleneinrichtung insbesondere dadurch, dass eine zweite Laserdiode 85 und ein Spiegel 94 vorgesehen sind. Die zweite Laserdiode 85 und der Spiegel 94 sind so angeordnet, dass ein von der zweiten Laserdiode 85 erzeugtes drittes Lichtbündel von dem Objektiv 92 ebenfalls in die Öffnung 74 in der Leuchtdiode 71 gebündelt wird. Damit können gleichzeitig oder alternierend das von der Leuchtdiode 71 erzeugte erste Lichtbündel, das von den ersten Laserdiode 81 erzeugte zweite Lichtbündel und das von den zweiten Laserdiode 85 erzeugte dritte Lichtbündel in den Beleuchtungsstrahlengang eingekoppelt werden.

Die erste Laserdiode 81 und die zweite Laserdiode 85 können ausgebildet sein, um Licht unterschiedlicher Wellenlängen zu emittieren. In diesem Fall ist der Spiegel 94 insbesondere dichroitisch, um Licht der ersten Laserdiode 81 überwiegend zu transmittieren und Licht der zweiten Laserdiode 85 überwiegend zu reflektieren. Mögliche Anwendungen und Vorteile entsprechen den oben anhand der Figuren 5 und 6 beschriebenen.

Abhängig von der Abstrahlungscharakteristik der Laserdioden 81, 85 kann es vorteilhaft sein, die von ihnen erzeugten Lichtbündel nicht zunächst in Lichtwellenleiter einzukoppeln, sondern, wie in den Figuren 8 und 9 gezeigt, mittels einer Linse oder eines Objektivs 92 eine Taille des bzw. der von den Laserdioden 81, 85 erzeugten Lichtbündel in der Öffnung 74 in der Leuchtdiode 71 zu erzeugen. Abweichend von der Darstellung in den Figuren 8 und 9 kann aber zusätzlich jeweils ein Lichtwellenleiter zur Übertragung des bzw. der von den Laserdioden 81, 85 erzeugten zweiten bzw. dritten Lichtbündel zu der Linse bzw. dem Objektiv 92 vorgesehen sein. Ferner können alternativ die von den Laserdioden 81, 85 erzeugte Lichtbündel zunächst mittels eines (insbesondere dichroitischen) Spiegels vereint und dann mittels eines Lichtwellenleiters zu dem Objektiv 92 übertragen werden. Ferner können über mehrere, insbesondere dichroitische, Spiegel von mehr als zwei Laserdioden erzeugte Lichtbündel zusammengeführt werden. Lichtquelleneinrichtungen ähnlich den in den Figuren 8 und 9 gezeigten Lichtquelleneinrichtungen können zur Erzeugung von Taillen bzw. Einschnürungen von einem oder mehreren von Laserdioden erzeugten Lichtbündeln in mehreren Öffnungen in der Leuchtdiode 71 oder an einem oder mehreren Orten am Rand der Leuchtdiode 71 ausgebildet sein. Eine Einkopplung von einem oder mehreren von Laserdioden erzeugten Lichtbündeln in den Beleuchtungsstrahlengang am Rand der Leuchtdiode 71 kann Vorteile haben, die oben mit Bezug auf die Figuren 4 und 6 beschrieben wurden.

Figur 10 zeigt eine schematische Darstellung einer nicht erfindungsgemäßen Variante der Lichtquelleneinrichtung 60, die in einigen Merkmalen den oben anhand der Figuren 2 bis 9 dargestellten Lichtquelleneinrichtungen ähnlich ist, insbesondere den oben anhand der Figuren 4, 7 und 8 dargestellten Lichtquelleneinrichtungen. Ähnlich wie bei den oben anhand der Figuren 4 und 7 dargestellten Lichtquelleneinrichtungen weist die Leuchtdiode 71 keine Öffnung auf. Zur Einkopplung eines von der Laserdiode 81 erzeugten zweiten Lichtbündels in den Beleuchtungsstrahlengang sind ein Spiegel 94 und, ähnlich wie oben anhand der Figuren 8 und 9 dargestellt, ein Objektiv 92 vorgesehen. Der Spiegel 94 ist im Lichtleitkörper 73 angeordnet, beispielsweise eingegossen, und kann dichroitisch sein, um von der Leuchtdiode 71 erzeugtes Licht überwiegend zu transmittieren und von der Laserdiode 81 erzeugtes Licht überwiegend zu reflektieren.

Die Laserdiode 81, das Objektiv 92 und der Spiegel 94 sind so angeordnet, dass das von der Laserdiode 81 erzeugte zweite Lichtbündel lichtstromabwärts des Spiegels 94 bzw. ab seiner Reflexion am Spiegel 94 im Beleuchtungsstrahlengang und im Wesentlichen parallel zu dem von der Leuchtdiode 71 erzeugten Lichtbündel verläuft. Damit bildet der Spiegel 94 bzw. dessen reflektierende Schicht den Ort der Einkopplung. Ab dem Ort der Einkopplung entspricht die Hauptstrahlungsrichtung des von der Laserdiode 81 erzeugten zweiten Lichtbündels im Wesentlichen der Hauptstrahlungsrichtung des von der Leuchtdiode 71 erzeugten ersten Lichtbündels. Die von der Leuchtdiode 71 und der Laserdiode 81 erzeugten Lichtbündel können sich jedoch auch lichtstromabwärts des Orts der Einkopplung in der Divergenz, der Intensitätsverteilung über den Querschnitt des Beleuchtungsstrahlengangs und in anderen Eigenschaften unterscheiden.

Abweichend von der Darstellung in Figur 10 kann zwischen der Laserdiode 81 und der Linse bzw. dem Objektiv 92 ein Lichtwellenleiter vorgesehen sein. Ferner können mit Hilfe eines oder mehrerer weiterer Spiegel und/oder eines oder mehrerer Linsen oder Objektive von einer oder mehreren weiteren Laserdioden erzeugte Lichtbündel in den Beleuchtungsstrahlengang eingekoppelt werden. Von mehreren Laserdioden erzeugte Lichtbündel können, ähnlich wie oben anhand der Figur 9 dargestellt, vor der Einkopplung in den Beleuchtungsstrahlengang zusammengeführt bzw. gemischt werden oder mittels mehrerer Spiegel im Lichtleitkörper 73 separat voneinander eingekoppelt werden.

Die Laserdiode 81, das Objektiv 92 und der Spiegel 94 können so angeordnet sein, dass das von der Laserdiode 81 erzeugte zweite Lichtbündel am Ort der Einkopplung bzw. am Spiegel 94 eine Taille bzw. Einschnürung aufweist. Die Größe bzw. Ausdehnung des Spiegels 94 kann an den Querschnitt des von der Laserdiode 81 erzeugten zweite Lichtbündels am Ort der Einschnürung angepasst sein. Der Spiegel 94 kann in diesem Fall sehr klein, insbesondere kleiner oder deutlich kleiner als ein Millimeter sein. Insbesondere wenn die Leuchtdiode 71 und die Laserdiode 81 Licht der gleichen Wellenlänge emittieren, kann auf diese Weise die Abschattung des von der Leuchtdiode 71 erzeugten ersten Lichtbündels durch den Spiegel 94 auf ein Minimum reduziert werden.

Figur 11 zeigt eine schematische Darstellung einer weiteren, nicht erfindungsgemäßen Variante der Lichtquelleneinrichtung 60, die in mehreren Merkmalen der oben anhand der Figur 10 dargestellten Lichtquelleneinrichtung ähnlich ist. Anders als bei der oben anhand der Figur 10 dargestellten Lichtquelleneinrichtung ist ein Lichtwellenleiter 82 zur Übertragung eines von der Laserdiode 81 erzeugten zweiten Lichtbündels vorgesehen, dessen erstes Ende 83 mit der Laserdiode 81 gekoppelt ist. Zwischen dem zweiten Ende 84 des Lichtwellenleiters 81 und dem Lichtleitkörper 73 ist eine Gradientenindexlinse 95 angeordnet, um das von der Laserdiode 81 erzeugte und mittels des Lichtwellenleiters 82 übertragene zweite Lichtbündel auf den Spiegel 94 im Lichtleitkörper 73 zu kollimieren. Die Länge des Lichtleitkörpers 73 in Längsrichtung des Beleuchtungsstrahlengangs bzw. senkrecht zur lichtemittierenden Fläche 72 der Leuchtdiode 71 ist kürzer als bei der oben anhand der Figur 10 dargestellten Lichtquelleneinrichtung, kann jedoch ebenso lang sein, wie bei jener. Umgekehrt kann auch bei der oben anhand der Figur 10 dargestellten Lichtquelleneinrichtung der Lichtleitkörper so kurz wie bei der Lichtquelleneinrichtung 60 aus Figur 11 sein.

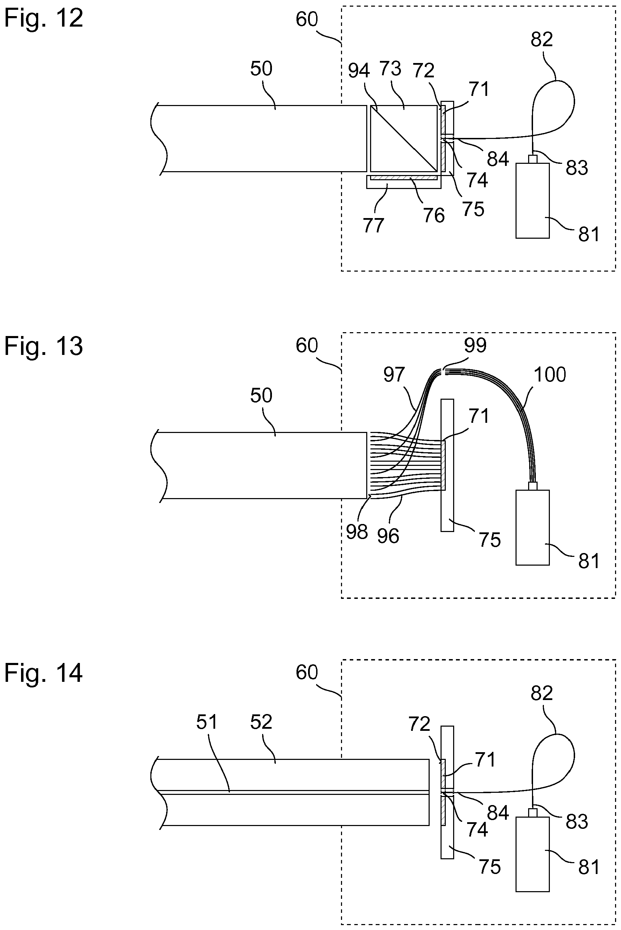

Figur 12 zeigt eine schematische Darstellung einer Lichtquelleneinrichtung 60, die in einigen Merkmalen der oben anhand der Figur 2 dargestellten Lichtquelleneinrichtung ähnlich ist. Am Lichtleitkörper 73 ist eine zweite Leuchtdiode 76 mit einem zweiten Kühlkörper 77 angeordnet. Im Lichtleitkörper 73 ist ein Spiegel 94 angeordnet, der insbesondere dichroitisch ist. Die zweite Leuchtdiode 76 und der Spiegel 94 sind so angeordnet, dass ein von der zweiten Leuchtdiode 76 erzeugtes Lichtbündel mittels des Spiegels 94 in den Beleuchtungsstrahlengang eingekoppelt wird. Ähnlich wie bei den oben anhand der Figuren 10 und 11 dargestellten Lichtquelleneinrichtungen stellt somit der Spiegel 94 bzw. dessen reflektierende Schicht den Ort der Einkopplung des von der zweiten Leuchtdiode 76 erzeugten Lichtbündels dar. Ab dem Ort der Einkopplung bzw. dem Spiegel 94 lichtstromabwärts verläuft das von der zweiten Leuchtdiode 76 erzeugte Lichtbündel im Beleuchtungsstrahlengang und im Wesentlichen parallel zu und in der gleichen Richtung wie das von der ersten Leuchtdiode 71 erzeugte Lichtbündel und das von der Laserdiode 81 erzeugte und am zweiten Ende 84 des Lichtwellenleiters 82 in den Beleuchtungsstrahlengang eingekoppelte Lichtbündel.

Beispielsweise ist eine der beiden Leuchtdioden 71, 76 eine Weißlicht-Leuchtdiode bzw. eine weiße Leuchtdiode, die zur Erzeugung von Licht mit einem Spektrum, das vom menschlichen Auge als weiß wahrgenommen wird, ausgebildet ist. Die jeweils andere Leuchtdiode ist beispielsweise zur Erzeugung von Licht im blauen oder violetten Spektralbereich ausgebildet, das zur Anregung von Fluoreszenz geeignet ist. Die Laserdiode 81 ist beispielsweise ebenfalls zur Emission von zur Anregung von Fluoreszenz geeignetem Licht oder zur Korrektur bzw. Ergänzung des von der weißen Leuchtdiode erzeugten Spektrums ausgebildet.

Ähnlich wie bereits oben mit Bezug auf andere Figuren erwähnt, kann auch die in Figur 2 gezeigte Lichtquelleneinrichtung 60 mehr als zwei Leuchtdioden und/oder mehrere Laserdioden 81 umfassen. Zur Vereinigung bzw. Mischung der von drei oder mehr Leuchtdioden erzeugten Lichtbündel können im Lichtleitkörper 73 und/oder außerhalb desselben zwei bzw. mehr Spiegel vorgesehen sein, die jeweils insbesondere dichroitisch sind. Beispielsweise können Leuchtdioden unmittelbar an bis zu fünf Seiten eines quaderförmigen Lichtleitkörpers 73 angeordnet sein. Zusätzlich können von einer oder mehreren Laserdioden erzeugte Lichtbündel, wie oben anhand der Figuren 2 bis 11 dargestellt, eingekoppelt werden.

Figur 13 zeigt eine schematische Darstellung einer weiteren, nicht erfindungsgemäßen Variante der Lichtquelleneinrichtung 60 mit einer Leuchtdiode 71 an einem Kühlkörper 75 und einer Laserdiode 81. Erste Lichtwellenleiter 96 koppeln die Leuchtdiode 71 mit einer Lichteintrittsfläche eines Lichtleitkabels 50. Zweite Lichtwellenleiter 97 koppeln eine Koppelstelle 99 mit der Lichteintrittsfläche des Lichtleitkabels 50. Die zweiten Enden der ersten Lichtwellenleiter 96 und die zweiten Enden der zweiten Lichtwellenleiter 97 sind parallel zueinander an einer Fläche 98 angeordnet, die der Lichteintrittsfläche des Lichtleitkabels 50 gegenüberliegt oder mit dieser identisch ist.

Einer oder mehrere dritte Lichtwellenleiter 100 koppeln die Laserdiode 81 mit der Koppelstelle 99. An der Koppelstelle 99 können die zweiten Lichtwellenleiter 97 und der oder die dritten Lichtwellenleiter 100 mittels einer Steckverbindung oder einer anderen Kupplung mechanisch und optisch gekoppelt bzw. verbunden sein. Zur optischen Kopplung kann optional eine in Figur 13 nicht dargestellte Linse oder eine andere optische Einrichtung vorgesehen sein. Alternativ können an der Koppelstelle Stirnflächen der zweiten Lichtwellenleiter 97 einerseits und des oder der dritten Lichtwellenleiter 100 andererseits unmittelbar aneinander grenzen. Alternativ können die zweiten Lichtwellenleiter 97 abweichend von der Darstellung in Figur 13 unmittelbar mit der Laserdiode 81 verbunden sein.

Die ersten Lichtwellenleiter 96 oder die ersten Lichtwellenleiter 96 und das Lichtleitkabel 50 definieren einen Beleuchtungsstrahlengang zur Bereitstellung eines von der Leuchtdiode 71 erzeugten ersten Lichtbündels für eine endoskopische oder exoskopische Anwendung. Die zweiten Lichtwellenleiter 97 bilden eine Einkopplungseinrichtung zum Einkoppeln des von der Laserdiode 81 erzeugten zweiten Lichtbündels in den Beleuchtungsstrahlengang. Spätestens ab der Fläche 98 verläuft das von der Laserdiode 81 erzeugte zweiten Lichtbündel im Beleuchtungsstrahlengang und im Wesentlichen parallel zu dem von der Leuchtdiode 71 erzeugten ersten Lichtbündel. Der Ort der Einkopplung liegt deshalb an der Fläche 98 oder lichtstromaufwärts derselben, wenn die zweiten Lichtwellenleiter 97 bereits lichtstromaufwärts der Fläche 98 parallel zu den ersten Lichtwellenleitern 96 angeordnet sind.

Die zweiten Enden der ersten Lichtwellenleiter 96 und die zweiten Enden der zweiten Lichtwellenleiter 97 können jeweils zufällig, quasizufällig oder regelmäßig, insbesondere entsprechend einem ein- oder zweidimensionalen Gitter, an der Fläche 98 angeordnet sein. Die zweiten Enden der Lichtwellenleiter 96, 97 können mit der Lichteintrittsfläche des Lichtleitkabels 50 fest verbunden, insbesondere verkittet, sein. Alternativ liegen die zweiten Enden der Lichtwellenleiter 96, 97 und die Fläche 98 der Lichteintrittsfläche des Lichtleitkabels 50 mit einem Abstand gegenüber oder liegen an dieser an.

Die Summe der einzelnen Querschnittesflächen der lichtleitenden Kerne der zweiten Lichtwellenleiter 97 ist kleiner als die Summe der einzelnen Querschnittsflächen der lichtleitenden Kerne der ersten Lichtwellenleiter 96. Deshalb ist die gesamte Querschnittsfläche des von der Laserdiode 81 erzeugten zweiten Lichtbündels kleiner als die gesamte Querschnittsfläche des von der Leuchtdiode 71 erzeugten ersten Lichtbündels.

Abweichend von Figur 13 kann das von der Laserdiode 81 erzeugte zweite Lichtbündel über lediglich einen Lichtwellenleiter in den Beleuchtungsstrahlengang eingekoppelt werden. Abweichend von der Darstellung in Figur 13 können von mehr als einer Leuchtdiode 71 und/oder von mehr als einer Laserdiode 81 erzeugte zweite Lichtbündel mittels entsprechend angeordneter Lichtwellenleiter gemischt bzw. vereinigt werden. Abweichend von Figur 13 können die ersten und zweiten Lichtwellenleiter 96, 97 selbst ab dem Ort der Einkopplung als Lichtleitkabel weitergeführt sein, beispielsweise bis zu einer Kupplung, die mit dem oben anhand der Figur 1 dargestellten proximalen Ende 21 eines Endoskops 20 lösbar verbunden werden kann.

Wie bereits mehrfach erwähnt, können Merkmale der oben anhand der Figuren 2 bis 13 dargestellten Lichtquelleneinrichtungen teilweise miteinander kombiniert werden. Ferner können bei den oben anhand der Figuren 2 bis 13 dargestellten Lichtquelleneinrichtungen Spiegel durch optische Gitter ersetzt werden, insbesondere wenn sie zur Reflexion von im Wesentlichen monochromatischer Laserstrahlung vorgesehen sind. Wenn anstelle dichroitischer Spiegel solche mit von der Polarisation abhängiger Reflexion verwendet werden, können Lichtbündel entsprechender Polarisation auch bei gleicher Wellenlänge verlustfrei oder verlustarm vereint werden.

Wie bereits erwähnt, können bei den meisten der oben anhand der Figuren 2 bis 14 dargestellten Lichtquelleneinrichtungen jeweils anstelle einer Leuchtdiode mehrere Leuchtdioden bzw. ein Array von Leuchtdioden vorgesehen sein. Die Leuchtdioden eines Arrays können Licht mit gleichen Spektren oder mit verschiedenen Spektren erzeugen. Die Leuchtdioden können durch andere, insbesondere flächige, Lichtquellen ersetzt werden. Die Laserdioden können durch andere Laser oder durch andere Lichtquellen ersetzt werden. Beispielsweise können die Laserdioden durch Leuchtdioden, die mit einem photonischen Gitter kombiniert sind oder ein photonisches Gitter aufweisen, ersetzt werden.

Abweichend von den obigen Darstellungen anhand der Figuren 1 bis 13 können die Lichtquelleneinrichtungen nicht als separate Einheiten oder Geräte vorgesehen, sondern teilweise oder vollständig in ein Endoskop oder ein Exoskop integriert sein.

Figur 14 zeigt eine schematische Darstellung einer Lichtquelleneinrichtung 60, die in einigen Merkmalen der oben anhand der Figur 3 dargestellten Lichtquelleneinrichtungen ähnlich ist. Anders als bei der oben anhand der Figur 3 dargestellten Lichtquelleneinrichtung ist eine koaxiale Anordnung aus einem inneren Lichtwellenleiter 51 und einem äußeren Lichtwellenleiter 52 vorgesehen. Der innere Lichtwellenleiter 51 weist einen geringen Querschnitt auf und ist gegenüber der Öffnung 74 in der Leuchtdiode 71 angeordnet. Der äußere Lichtwellenleiter 52 weist einen ringförmigen Querschnitt auf, der zumindest näherungsweise der lichtemittierenden Fläche 72 der Leuchtdiode 71 entspricht und ist dieser gegenüber angeordnet.

Zwischen der lichtemittierenden Fläche 72 der Leuchtdiode 71 und der Lichteintrittsfläche der koaxialen Anordnung des inneren Lichtwellenleiters 51 und des äußeren Lichtwellenleiters 52 ist ein Zwischenraum vorgesehen. Dieser Zwischenraum bildet einen gemeinsamen Beleuchtungsstrahlengang, in dem das von der Leuchtdiode 71 erzeugte Lichtbündel und das von der Laserdiode 81 erzeugte Lichtbündel sich teilweise mischen.

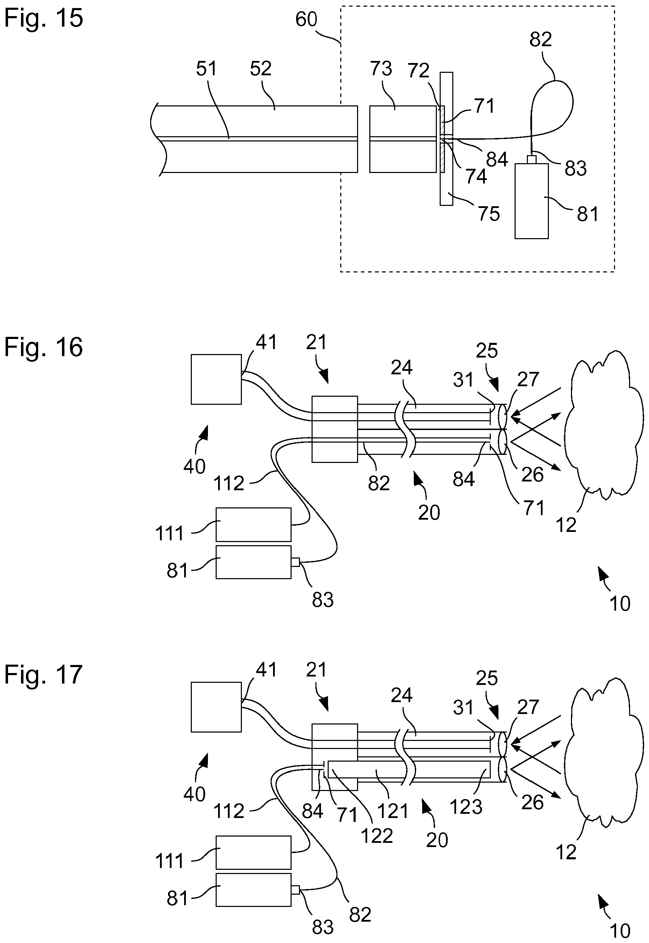

Figur 15 zeigt eine schematische Darstellung einer Lichtquelleneinrichtung 60, die in einigen Merkmalen den oben anhand der Figuren 3 und 14 dargestellten Lichtquelleneinrichtungen ähnlich ist. Abweichend von der oben anhand der Figur 14 dargestellten Lichtquelleneinrichtung ist zwischen der Leuchtdiode 71 und dem zweiten Ende 84 des Lichtwellenleiters 82 einerseits und der Lichteintrittsfläche der koaxialen Anordnung des inneren Lichtwellenleiters 51 und des äußeren Lichtwellenleiters 52 andererseits ein Lichtleitkörper 73 vorgesehen. Der Lichtleitkörper 73 weist jedoch anders als beispielsweise bei den oben anhand der Figuren 2 und 4 bis 9 dargestellten Beispielen einen Querschnitt auf, der zumindest im wesentlichen dem Querschnitt der koaxialen Anordnung des inneren Lichtwellenleiters 51 und des äußeren Lichtwellenleiters 52 entspricht.

Zumindest entweder zwischen der lichtemittierenden Fläche 72 der Lichtquelle 71 und der Lichteintrittsfläche des Lichtleitkörpers 73 oder zwischen der Lichtaustrittsfläche des Lichtleitkörpers 73 und der Lichteintrittsfläche der koaxialen Anordnung des inneren Lichtwellenleiters 51 und des äußeren Lichtwellenleiters 52 ist ein Zwischenraum vorgesehen. Dieser Zwischenraum bildet einen gemeinsamen Beleuchtungsstrahlengang, in dem das von der Leuchtdiode 71 erzeugte Lichtbündel und das von der Laserdiode 81 erzeugte Lichtbündel sich teilweise mischen. Alternativ kann bei den anhand der Figuren 14 und 15 dargestellten Lichtquelleneinrichtungen der Zwischenraum entfallen.

Figur 16 zeigt eine schematische Darstellung eines Beispiels eines Endoskopiesystems 10 mit einem Endoskop 20, das in einigen Merkmalen dem oben anhand der Figur 1 dargestellten Endoskop ähnlich ist. Abweichend von dem oben anhand der Figur 1 dargestellten Endoskopiesystem ist eine Leuchtdiode 71 am distalen Ende 25 des Endoskops 20 angeordnet. Eine Laserdiode 81, die außerhalb des Endoskops 20 angeordnet ist, ist über einen Lichtwellenleiter 82 mit dem distalen Ende 25 des Endoskops 20 gekoppelt. Ein erstes Ende 83 des Lichtwellenleiters 82 ist mit der Laserdiode 81 gekoppelt, ein zweites Ende 84 des Lichtwellenleiters 82 ist in einer Öffnung in der Leuchtdiode 71 angeordnet.

Eine Leistungsversorgungseinrichtung 111 ist über eine Leistungsversorgungsleitung 112 mit der ersten Leuchtdiode 71 am distalen Ende 25 des Endoskops 20 verbunden. Die Leistungsversorgungseinrichtung 111 ist zur Versorgung der Leuchtdiode 71 mit elektrischer Leistung vorgesehen. Zusätzlich kann die Leistungsversorgungseinrichtung 111 zur Leistungsversorgung der Laserdiode 81 vorgesehen sein. Die Leistungsversorgungseinrichtung 111 und die Laserdiode 81 können in einem Gerät oder in einer Einheit integriert sein. Der Lichtwellenleiter 82 und die Leistungsversorgungsleitung 112 können in einem Kabel integriert sein, zumindest zwischen der Laserdiode 81, der Leistungsversorgungseinrichtung 111 bzw. einem Geräts, das beide integriert, einerseits und dem Endoskop 20 andererseits. An der Laserdiode 81, der Leistungsversorgungseinrichtung 111 und/oder dem Endoskop 20 können Steckverbindungen oder andere Kupplungen vorgesehen sein, damit diese von dem Lichtwellenleiter 82, der Leistungsversorgungsleitung 112 bzw. dem beide integrierenden Kabel getrennt werden können.

Der Raumbereich am distalen Ende 25 des Endoskops 20 lichtstromabwärts der Leuchtdiode 71 bis zum Lichtaustrittsfenster 26 und einschließlich desselben bildet einen Beleuchtungsstrahlengang zur Bereitstellung von Beleuchtungslicht zur Beleuchtung des Objekts 12. Die Lichtaustrittsfläche am zweiten Ende 84 des Lichtwellenleiters 82 bildet den Ort der Einkopplung, ab dem lichtstromabwärts das von der Laserdiode 81 erzeugte und mittels des Lichtwellenleiters 82 übertragene zweite Lichtbündel in den Beleuchtungsstrahlengang eingekoppelt ist. Ab dem Ort der Einkopplung verläuft das von der Laserdiode 81 erzeugte zweite Lichtbündel im Beleuchtungsstrahlengang und im Wesentlichen parallel zu dem von der Leuchtdiode 71 erzeugten ersten Lichtbündel.

Abweichend von der Darstellung in Figur 14 können zahlreiche oben anhand der Figuren 2 bis 13 dargestellte Lichtquelleneinrichtungen teilweise oder vollständig in ein Endoskop integriert sein. Dabei können die Leuchtdioden und/oder die Laserdioden jeweils am distalen Ende 25 oder am proximalen Ende 21 des Endoskops 20 angeordnet sein. Insbesondere mittels der oben anhand der Figuren 2 bis 7 und 11 bis 13 beschriebenen Lichtwellenleiter 82, 97 können von räumlich beabstandeten Laserdioden erzeugte Lichtbündel in den Beleuchtungsstrahlengang eingekoppelt werden.

Figur 17 zeigt eine schematische Darstellung eines weiteren Beispiels eines Endoskopiesystems 10, das dem oben anhand der Figur 16 dargestellten Endoskopiesystem ähnlich ist. Abweichend von dem oben anhand der Figur 16 dargestellten Endoskopiesystem ist bei dem in Figur 17 gezeigten Endoskopiesystem 10 die Leuchtdiode 71 nicht am distalen Ende 25 sondern am proximalen Ende 21 des Endoskops 20 angeordnet. Ähnlich wie bei dem oben anhand der Figur 16 dargestellten Endoskopiesystem ist aber auch hier ein zweites Ende 84 des Lichtwellenleiters 82 in einer Öffnung in der Leuchtdiode 71 angeordnet.

Ein weiterer Lichtwellenleiter 121 ist im Schaft 24 des Endoskops 20 angeordnet. Ein erstes, proximales Ende 122 des weiteren Lichtwellenleiters 121 ist am proximalen Ende 21 des Endoskops gegenüber der Leuchtdiode 71 und dem zweiten Ende 84 des Lichtwellenleiters 82 oder angrenzend an diese angeordnet oder mit diesen dauerhaft verbunden, beispielsweise verkittet. Ein zweites, distales Ende 123 des weiteren Lichtwellenleiters 121 ist am distalen Ende 25 des Endoskops 20 am Lichtaustrittsfenster 26 angeordnet. Das von der Leuchtdiode 71 erzeugte Lichtbündel und das von der Laserdiode 81 erzeugte Lichtbündel werden gemeinsam von dem weiteren Lichtwellenleiter 121 vom proximalen Ende 21 zum distalen Ende 25 des Endoskops 20 übertragen, wo sie gemeinsam durch das Lichtaustrittsfenster 26 austreten.

Bei beiden oben anhand der Figuren 16 und 17 dargestellten Endoskopiesystemen kann das proximale Ende 21 des Endoskops 20 mit einem Gerät bzw. einer Einheit, welche die Laserdiode 81 und die Leistungsversorgung 111 integriert, über ein Kabel verbunden sein, in dem der Lichtwellenleiter 82 und die Leistungsversorgungsleitung 112 integriert sind. Der Lichtwellenleiter 82 und damit auch das Kabel können besonders dünn und flexibel sein.

### Bezugszeichen

- 10: Endoskopiesystem
- 12: Objekt
- 20: Endoskop
- 21: proximales Ende des Endoskops 20
- 22: Kupplung für Lichtleitkabel 60
- 23: Kupplung für Kamera 30
- 24: Schaft des Endoskops 20
- 25: distales Ende des Endoskops 20
- 26: Lichtaustrittsfenster am distalen Ende 25 des Endoskops 20
- 27: Lichteintrittsfenster am distalen Ende 25 des Endoskops 20
- 30: Kamera
- 31: lichtempfindlicher Bildsensor der Kamera 30
- 32: Objektiv der Kamera 30
- 40: Kamerasteuerung
- 41: Shuttersignalausgang der Kamerasteuerung 40
- 50: Lichtleitkabel
- 51: innerer Lichtwellenleiter des Lichtleitkabels 50
- 52: äußerer Lichtwellenleiter des Lichtleitkabels 50
- 60: Lichtquelleneinrichtung
- 71: erste Leuchtdiode oder erstes Array von Leuchtdioden
- 72: lichtemittierende Fläche der ersten Leuchtdiode 71
- 73: Lichtleitkörper
- 74: Öffnung in der ersten Leuchtdiode 71
- 75: Kühlkörper der ersten Leuchtdiode 71
- 76: zweite Leuchtdiode oder zweites Array von Leuchtdioden
- 77: Kühlkörper der zweiten Leuchtdiode 76
- 81: erste Laserdiode
- 82: erster Lichtwellenleiter
- 83: erstes Ende des ersten Lichtwellenleiters 82
- 84: zweites Ende des ersten Lichtwellenleiters 82
- 85: zweite Laserdiode
- 86: zweiter Lichtwellenleiter
- 87: erstes Ende des zweiten Lichtwellenleiters 86
- 88: zweites Ende des zweiten Lichtwellenleiters 86
- 91: Linse in der Öffnung 74
- 92: Sammellinse
- 94: dichroitischer Spiegel
- 95: Gradientenindexlinse
- 96: erster Lichtwellenleiter
- 97: zweiter Lichtwellenleiter
- 98: Fläche
- 99: Koppelstelle zwischen den zweiten Lichtwellenleitern 97 und den dritten Lichtwellenleitern 99
- 100: dritter Lichtwellenleiter
- 111: Leistungsversorgungseinrichtung für die erste Leuchtdiode 71
- 112: Leistungsversorgungsleitung für die erste Leuchtdiode 71
- 121: weiterer Lichtwellenleiter
- 122: erstes Ende des weiteren Lichtwellenleiters 101
- 123: zweites Ende des weiteren Lichtwellenleiters 101

## Patentansprüche

1. Lichtquelleneinrichtung (60) für endoskopische oder exoskopische Anwendungen, mit:
einer flächigen ersten Lichtquelle (71) zum Erzeugen von Licht;
einer zweiten Lichtquelle (81) zum Erzeugen von Licht;
einem Beleuchtungsstrahlengang (73, 50; 96; 26), der dazu ausgebildet ist, ein von der ersten Lichtquelle (71) ausgehendes erstes Lichtbündel für eine endoskopische oder exoskopische Anwendung bereitzustellen;
einer Einkopplungseinrichtung (82, 91; 92; 94, 95; 97) zum Einkoppeln eines zweiten Lichtbündels von der zweiten Lichtquelle (81) in den Beleuchtungsstrahlengang (73, 50; 96; 26), wobei die Einkopplungseinrichtung (82, 91; 92; 94, 95; 97) so ausgebildet ist, dass am Ort der Einkopplung die Querschnittsfläche des zweiten Lichtbündels kleiner ist als die Querschnittsfläche des ersten Lichtbündels und dass der Ort der Einkopplung in der lichtemittierenden Fläche oder in einer ebenen oder minimal gekrümmten Fläche, welche die lichtemittierende Fläche der ersten Lichtquelle enthält, liegt,
wobei die erste Lichtquelle (71)
entweder eine Halbleiterlichtquelle mit einem Array von Leuchtdioden oder ein Array von anderen lichtemittierenden Elementen umfasst und der Ort der Einkopplung zwischen Leuchtdioden bzw. anderen lichtemittierenden Elementen des Arrays angeordnet ist, oder die erste Lichtquelle eine Leuchtdiode (71) mit einer Öffnung (74) umfasst, wobei der Ort der Einkopplung an der Öffnung (74) angeordnet ist,
und wobei jeweils
die Einkopplungseinrichtung einen Lichtwellenleiter (82; 97) mit einem ersten Ende (83) und einem zweiten Ende (84) umfasst, wobei das erste Ende (82) des Lichtwellenleiters (92; 97) mit der zweiten Lichtquelle (81) gekoppelt ist, und wobei das zweite Ende (84) des Lichtwellenleiters (92; 97) in oder neben der lichtemittierenden Fläche (72) der ersten Lichtquelle (71) angeordnet ist,
oder
die Einkopplungseinrichtung zumindest entweder ein Objektiv (91; 92), einen gekrümmten Spiegel, ein optisches Gitter oder eine andere abbildende Einrichtung zum Erzeugen einer Taille des zweiten Lichtbündels am Rand der lichtemittierenden Fläche (72) der ersten Lichtquelle (71) oder in einer Öffnung (74) in der lichtemittierenden Fläche (72) der ersten Lichtquelle (71) umfasst.

2. Lichtquelleneinrichtung (60) nach dem vorangehenden Anspruch, bei der die Querschnittsfläche des zweiten Lichtbündels nicht mehr als die Hälfte der Querschnittsfläche des ersten Lichtbündels beträgt.

3. Lichtquelleneinrichtung (60) nach einem der vorangehenden Ansprüche, bei der die erste Lichtquelle zur Erzeugung eines ersten Spektrums mit einer ersten Halbwertsbreite ausgebildet ist, und bei dem die zweite Lichtquelle zur Erzeugung eines zweiten Spektrums mit einer zweiten Halbwertsbreite ausgebildet ist, wobei die zweite Halbwertsbreite nicht mehr als die Hälfte der ersten Halbwertsbreite beträgt.

4. Lichtquelleneinrichtung (60) nach einem der vorangehenden Ansprüche, bei der die zweite Lichtquelle eine Laserdiode (81), einen anderen Diodenlaser oder einen anderen Laser umfasst.

5. Lichtquelleneinrichtung nach einem der vorangehenden Ansprüche, bei der die erste Lichtquelle (71) in einem Endoskop (20) oder in einem Exoskop und die zweite Lichtquelle (81) separat von dem Endoskop (20) bzw. von dem Exoskop angeordnet ist.

## Claims

1. Light source device (60) for endoscopic or exoscopic applications, comprising:
a planar first light source (71) for generating light;
a second light source (81) for generating light;
an illumination beam path (73, 50; 96; 26) configured to provide a first beam of light emanating from the first light source (71) for endoscopic or exoscopic application;
a coupling means (82, 91; 92; 94, 95; 97) for coupling a second light beam from the second light source (81) into the illumination beam path (73, 50; 96; 26), wherein the coupling means (82, 91; 92; 94, 95; 97) is configured in such a way that, at the coupling site, the cross-sectional area of the second light beam is smaller than the cross-sectional area of the first light beam, and that the coupling site is located in the light-emitting surface or in a planar or minimally curved surface which contains the light-emitting surface of the first light source,
wherein the first light source (71)
either comprises a semiconductor light source with an array of light emitting diodes or an array of other light emitting elements and the coupling site is arranged between light emitting diodes or other light emitting elements of the array, respectively, or the first light source comprises a light emitting diode (71) with an opening (74), wherein the coupling site is located at the opening (74),
and wherein in each case
the coupling means comprises an optical waveguide (82; 97) having a first end (83) and a second end (84), wherein the first end (82) of the optical waveguide (92; 97) is coupled to the second light source (81), and wherein the second end (84) of the optical waveguide (92; 97) is located in or adjacent to the light emitting surface (72) of the first light source (71),
or
the coupling means comprises at least one of an objective (91; 92), a curved mirror, an optical grating or another imaging means for generating a waist of the second light beam at the edge of the light emitting surface (72) of the first light source (71) or in an opening (74) in the light emitting surface (72) of the first light source (71).

2. Light source device (60) according to the preceding claim, wherein the cross-sectional area of the second light beam is not more than half the cross-sectional area of the first light beam.

3. Light source device (60) according to any one of the preceding claims, wherein the first light source is configured to produce a first spectrum having a first value of full width at half maximum, and wherein the second light source is configured to produce a second spectrum having a second value of full width at half maximum, the second value being no more than half the first value.

4. Light source device (60) according to any one of the preceding claims, wherein the second light source comprises a laser diode (81), another diode laser or another laser.

5. Light source device according to any one of the preceding claims, wherein the first light source (71) is located in an endoscope (20) or in an exoscope and the second light source (81) is located separately from the endoscope (20) or from the exoscope.

## Revendications

1. Dispositif à sources de lumière (60) destiné à des applications endoscopiques ou exoscopiques, ledit dispositif comprenant :
une première source de lumière (71) sensiblement bidimensionnelle destinée à générer de la lumière ;
une deuxième source de lumière (81) destinée à générer de la lumière ;
un trajet de faisceau d'éclairage (73, 50 ; 96 ; 26) qui est conçu pour fournir un premier faisceau de lumière émanant de la première source de lumière (71) et destiné à une application endoscopique ou exoscopique ;
un dispositif d'injection par couplage (82, 91 ; 92 ; 94, 95 ; 97) destiné à injecter par couplage un deuxième faisceau de lumière de la deuxième source de lumière (81) dans le trajet du faisceau d'éclairage (73, 50 ; 96 ; 26), le dispositif d'injection par couplage (82, 91 ; 92 ; 94, 95 ; 97) étant conçu de telle manière que la section transversale du deuxième faisceau de lumière au site d'injection par couplage soit inférieure à la section transversale du premier faisceau de lumière et en ce que le site d'injection par couplage est situé dans la surface photoémettrice ou dans une surface plane ou légèrement incurvée qui contient la surface photoémettrice de la première source de lumière,
la première source de lumière (71) comprenant soit une source de lumière à semiconducteur pourvue d'un réseau de diodes électroluminescentes soit un réseau d'autres éléments photoémetteurs et le site d'injection par couplage étant disposé entre des diodes électroluminescentes ou d'autres éléments photoémetteurs du réseau, ou la première source de lumière comprenant une diode électroluminescente (71) pourvue d'une ouverture (74), le site d'injection par couplage étant disposé au niveau de l'ouverture (74),
et respectivement
le dispositif d'injection par couplage comprenant un guide d'ondes de lumière (82 ; 97) pourvu d'une première extrémité (83) et d'une deuxième extrémité (84), la première extrémité (82) du guide d'ondes de lumière (92 ; 97) étant couplée à la deuxième source de lumière (81), et la deuxième extrémité (84) du guide d'ondes de lumière (92 ; 97) étant disposée dans la surface photoémettrice (72) de la première source de lumière (71) ou à côté de celle-ci,
ou
le dispositif d'injection par couplage comprenant au moins un objectif (91 ; 92), un miroir incurvé, un réseau optique ou un autre dispositif de reproduction destiné à générer une taille du deuxième faisceau de lumière au bord de la surface photoémettrice (72) de la première source de lumière (71) ou dans une ouverture (74) ménagée dans la surface photoémettrice (72) de la première source de lumière (71).

2. Dispositif à sources de lumière (60) selon la revendication précédente, dans lequel la section transversale du deuxième faisceau de lumière n'est pas supérieure à la moitié de la section transversale du premier faisceau de lumière.

3. Dispositif à sources de lumière (60) selon l'une des revendications précédentes, dans lequel la première source de lumière est conçue pour générer un premier spectre ayant une première demi-largeur, et dans lequel la deuxième source de lumière est conçue pour générer un deuxième spectre ayant une deuxième demi-largeur, la deuxième demi-largeur ne dépassant pas plus de la moitié de la première demi-largeur.

4. Dispositif à sources de lumière (60) selon l'une des revendications précédentes, dans lequel la deuxième source de lumière comprend une diode laser (81), une autre diode laser ou un autre laser.

5. Dispositif à sources de lumière selon l'une des revendications précédentes, dans lequel la première source de lumière (71) est disposée dans un endoscope (20) ou dans un exoscope et la deuxième source de lumière (81) est disposée séparément de l'endoscope (20) ou de l'exoscope.
